(19) Europäisches Patentamt
European Patent Office
Office européen des brevets

(11) EP 2 283 018 B1

(12) # EUROPEAN PATENT SPECIFICATION

(45) Date of publication and mention of the grant of the patent:
**13.04.2016 Bulletin 2016/15**

(21) Application number: **09747582.6**

(22) Date of filing: **14.05.2009**

(51) Int Cl.:
***C07D 487/04*** *(2006.01)* ***A61K 31/407*** *(2006.01)*
***A61P 31/14*** *(2006.01)*

(86) International application number:
**PCT/US2009/043961**

(87) International publication number:
**WO 2009/140500 (19.11.2009 Gazette 2009/47)**

(54) **HEPATITIS C VIRUS INHIBITORS**

HEMMER DES HEPATITIS-C-VIRUS

INHIBITEURS DU VIRUS DE L'HÉPATITE C

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **15.05.2008 US 53489**

(43) Date of publication of application:
**16.02.2011 Bulletin 2011/07**

(73) Proprietor: **Bristol-Myers Squibb Company**
**Princeton, NJ 08543 (US)**

(72) Inventors:
- **SUN, Li-Qiang**
**Wallingford, CT 06492 (US)**
- **SCOLA, Paul, Michael**
**Wallingford, CT 06492 (US)**

(74) Representative: **Reitstötter Kinzebach**
**Patentanwälte**
**Sternwartstrasse 4**
**81679 München (DE)**

(56) References cited:
**WO-A-2004/094452**
**WO-A-2007/015824**
**WO-A-2007/056120**
**WO-A-2008/021960**
**WO-A-2008/137779**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 283 018 B1

## Description

The present disclosure is generally directed to antiviral compounds, and more specifically directed to compounds which inhibit the function of the NS3 protease (also referred to herein as "serine protease") encoded by Hepatitis C virus (HCV), compositions comprising such compounds, and methods for inhibiting the function of the NS3 protease.

HCV is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma.

Presently, the most effective HCV therapy employs a combination of alpha-interferon and ribavirin, leading to sustained efficacy in 40% of patients. Recent clinical results demonstrate that pegylated alpha-interferon is superior to unmodified alpha-interferon as monotherapy. However, even with experimental therapeutic regimens involving combinations of pegylated alpha-interferon and ribavirin, a substantial fraction of patients do not have a sustained reduction in viral load. Thus, there is a clear and unmet need to develop effective therapeutics for treatment of HCV infection.

HCV is a positive-stranded RNA virus. Based on a comparison of the deduced amino acid sequence and the extensive similarity in the 5' untranslated region, HCV has been classified as a separate genus in the Flaviviridae family. All members of the Flaviviridae family have enveloped virions that contain a positive stranded RNA genome encoding all known virus-specific proteins *via* translation of a single, uninterrupted, open reading frame.

Considerable heterogeneity is found within the nucleotide and encoded amino acid sequence throughout the HCV genome. Six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non- structural (NS) proteins. In the case of HCV, the generation of mature non-structural proteins (NS2, NS3, NS4A, NS4B, NS5A, and NS5B) is effected by two viral proteases. The first one cleaves at the NS2-NS3 junction; the second one is a serine protease contained within the *N*-terminal region of NS3 and mediates all the subsequent cleavages downstream of NS3, both in cis, at the NS3-NS4A cleavage site, and in trans, for the remaining NS4A- NS4B, NS4B-NS5A, NS5A-NS5B sites. The NS4A protein appears to serve multiple functions, acting as a co-factor for the NS3 protease and possibly assisting in the membrane localization of NS3 and other viral replicase components. The complex formation of the NS3 protein with NS4A is essential for efficient polyprotein processing, enhancing the proteolytic cleavage at all of the sites. The NS3 protein also exhibits nucleoside triphosphatase and RNA helicase activities. NS5B is a RNA-dependent RNA polymerase that is involved in the replication of HCV.

The prior art describes macrocyclic compounds useful for treating hepatitis C. For example, WO 2007/015824A and WO2007/056120A describe macrocyclic compounds inhibiting HCV. WO2004/094452A describes macrocyclic isoquinoline peptide inhibitors of HCV and WO 2008/021960 A describes triazolyl macrocyclic Hepatits C serine protease inhibitors. Further, WO 2008/137779A describes nacrocyclic HCV replication inhibitors.

The present invention relates to a compound of formula (I)

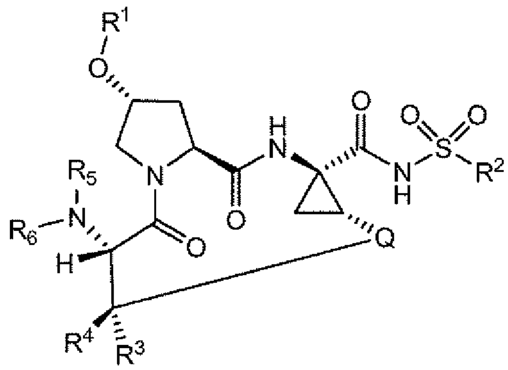

(I),

or a pharmaceutically acceptable salt thereof, wherein

Q is a $C_{3-9}$ saturated or unsaturated chain optionally containing from one to three heteroatoms independently selected

from O, $S(O)_m$, and $NR^8$; wherein m is 0, 1, or 2, and $R^8$ is selected from hydrogen, alkoxy, alkoxycarbonyl, alkyl, alkylcarbonyl, alkylsulfonyl, aminocarbonyl, arylsulfonyl, cycloalkyl, (cycloalkyl)alkyl, cycloalkyloxy, dialkylaminocarbonyl, dialkylaminocarbonylalkyl, haloalkyl, and hetero cyclyl carbonyl;

$R^1$ is isoquinolinyl optionally substituted with one or two $R^7$ groups;

$R^2$ is selected from alkyl, aryl, cycloalkyl, (cycloalkyl)alkyl, heterocyclyl, and $-NR^aR^b$, wherein the alkyl, the cycloalkyl and the cycloalkyl part of the (cycloalkyl)alkyl are optionally substituted with one, two, or three substituents selected from alkenyl, alkoxy, alkoxyalkyl, alkyl, arylalkyl, arylcarbonyl, cyano, cycloalkenyl, (cycloalkyl)alkyl, halo, haloalkoxy, haloalkyl, and $(NR^eR^f)$carbonyl;

$R^3$ and $R^4$ are independently selected from hydrogen, alkoxyalkyl, alkyl, haloalkoxyalkyl, and haloalkyl;

$R^5$ is selected from hydrogen, alkyl and haloalkyl;

$R^6$ is selected from phenyl and a five- or six-membered partially or fully unsaturated ring optionally containing one, two, three, or four heteroatoms selected from nitrogen, oxygen, and sulfur; wherein each of the rings is optionally substituted with one, two, three, or four substitutents independently selected from alkoxy, alkoxycarbonyl, alkyl, alkylcarbonyl, alkylsulfanyl, carboxy, cyano, cycloalkyl, cycloalkyloxy, halo, haloalkyl, haloalkoxy, $-NR^cR^d$, $(NR^eR^f)$carbonyl, $(NR^eR^f)$sulfonyl, and oxo; provided that when $R^6$ is a six-membered substituted ring all substituents on the ring other than fluoro must be in the meta and/or para positions relative to the ring's point of attachment to the parent molecular moiety;

each $R^7$ is independently selected from alkoxy, alkoxycarbonyl, alkyl, alkylcarbonyl, aryl, carboxy, cyano, cyanoalkyl, cycloalkyl, halo, haloalkyl, haloalkoxy, heterocyclyl, hydroxy, hydroxyalkyl, nitro,$-NR^cR^d$, $(NR^cR^d)$alkyl, $(NR^cR^d)$alkoxy, $(NR^eR^f)$carbonyl, and $(NR^eR^f)$sulfonyl; or

two adjacent $R^7$ groups, together with the carbon atoms to which they are attached, form a four- to seven-membered partially- or fully-unsaturated ring optionally containing one or two heteroatoms independently selected from nitrogen, oxygen, and sulfur, wherein the ring is optionally substituted with one, two, or three groups independently selected from alkoxy, alkyl, cyano, halo, haloalkoxy, and haloalkyl;

$R^a$ and $R^b$ are independently selected from hydrogen, alkoxy, alkyl, aryl, arylalkyl, cycloalkyl, (cycloalkyl)alkyl, heterocyclyl, and heterocyclylalkyl; or $R^a$ and $R^b$ together with the nitrogen atom to which they are attached form a four to seven-membered monocyclic heterocyclic ring;

$R^c$ and $R^d$ are independently selected from hydrogen, alkoxyalkyl, alkoxycarbonyl, alkyl, alkylcarbonyl, arylalkyl, and haloalkyl;

$R^e$ and $R^f$ are independently selected from hydrogen, alkyl, aryl, arylalkyl, and heterocyclyl; wherein the aryl, the aryl part of the arylalkyl, and the heterocyclyl are optionally substituted with one or two substituents independently selected from alkoxy, alkyl, and halo; and

$R^g$ and $R^h$ are independently selected from hydrogen, alkyl, aryl, arylalkyl, cycloalkyl, (cycloalkyl)alkyl, heterocyclyl, and heterocyclyl; or $R^g$ and $R^h$ together with the nitrogen atom to which they are attached form a monocyclic heterocyclic ring wherein the monocyclic heterocyclic ring is optionally fused to a phenyl ring to form a bicyclic system; wherein the monocyclic heterocyclic ring and the bicyclic system are optionally substituted with one, two, or three substituents independently selected from alkoxy, alkyl, halo, haloalkoxy, and haloalkyl.

The present invention further relates to a compound of formula (I)

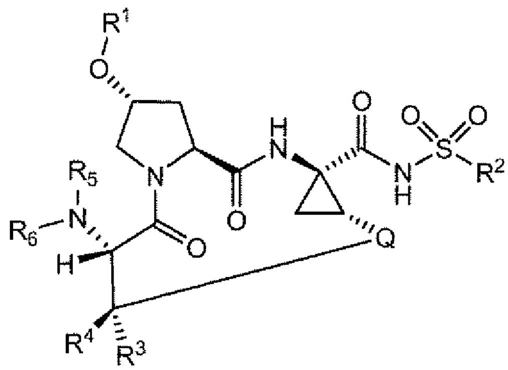

(I),

or a pharmaceutically acceptable salt thereof, wherein

Q is a $C_{3-9}$ saturated or unsaturated chain optionally containing from one to three heteroatoms independently selected from O, $S(O)_m$, and $NR^8$; wherein m is 0, 1, or 2, and $R^8$ is selected from hydrogen, alkoxy, alkoxycarbonyl, alkyl, alkylcarbonyl, alkylsulfonyl, aminocarbonyl, arylsulfonyl, cycloalkyl, (cycloalkyl)alkyl, cycloalkyloxy, dialkylaminocarbonyl, dialkylaminocarbonylalkyl, haloalkyl, and heterocyclylcarbonyl;

$R^1$ is isoquinolinyl optionally substituted with one or two $R^7$ groups;

$R^2$ is selected from alkyl, aryl, cycloalkyl, (cycloalkyl)alkyl, heterocyclyl, and $-NR^aR^b$, wherein the alkyl, the cycloalkyl and the cycloalkyl part of the (cycloalkyl)alkyl are optionally substituted with one, two, or three substituents selected from alkenyl, alkoxy, alkoxyalkyl, alkyl, arylalkyl, arylcarbonyl, cyano, cycloalkenyl, (cycloalkyl)alkyl, halo, haloalkoxy, haloalkyl, and $(NR^eR^f)$carbonyl;

$R^3$ and $R^4$ are independently selected from hydrogen, alkoxyalkyl, alkyl, haloalkoxyalkyl, and haloalkyl;

$R^5$ is selected from hydrogen, alkyl and haloalkyl;

$R^6$ is selected from phenyl and a five- or six-membered partially or fully unsaturated ring optionally containing one, two, three, or four heteroatoms selected from nitrogen, oxygen, and sulfur; wherein each of the rings is optionally substituted with one, two, three, or four substitutents independently selected from aryl; provided that when $R^6$ is a six-membered substituted ring all substituents on the ring other than fluoro must be in the meta and/or para positions relative to the ring's point of attachment to the parent molecular moiety;

each $R^7$ is independently selected from alkoxy, alkoxycarbonyl, alkyl, alkylcarbonyl, aryl, carboxy, cyano, cyanoalkyl, cycloalkyl, halo, haloalkyl, haloalkoxy, heterocyclyl, hydroxy, hydroxyalkyl, nitro,$-NR^cR^d$, $(NR^cR^d)$alkyl, $(NR^cR^d)$alkoxy, $(NR^eR^f)$carbonyl, and $(NR^eR^f)$sulfonyl; or

two adjacent $R^7$ groups, together with the carbon atoms to which they are attached, form a four- to seven-membered partially- or fully-unsaturated ring optionally containing one or two heteroatoms independently selected from nitrogen, oxygen, and sulfur, wherein the ring is optionally substituted with one, two, or three groups independently selected from alkoxy, alkyl, cyano, halo, haloalkoxy, and haloalkyl;

$R^a$ and $R^b$ are independently selected from hydrogen, alkoxy, alkyl, aryl, arylalkyl, cycloalkyl, (cycloalkyl)alkyl, heterocyclyl, and heterocyelylalkyl; or $R^a$ and $R^b$ together with the nitrogen atom to which they are attached form a four to seven-membered monocyclic heterocyclic ring;

$R^c$ and $R^d$ are independently selected from hydrogen, alkoxyalkyl, alkoxycarbonyl, alkyl, alkylcarbonyl, arylalkyl, and haloalkyl;

$R^e$ and $R^f$ are independently selected from hydrogen, alkyl, aryl, arylalkyl, and heterocyclyl; wherein the aryl, the aryl part of the arylalkyl, and the heterocyclyl are optionally substituted with one or two substituents independently selected from alkoxy, alkyl, and halo; and

$R^g$ and $R^h$ are independently selected from hydrogen, alkyl, aryl, arylalkyl, cycloalkyl, (cycloalkyl)alkyl, heterocyclyl, and heterocyclyl; or $R^g$ and $R^h$ together with the nitrogen atom to which they are attached form a monocyclic heterocyclic ring wherein the monocyclic heterocyclic ring is optionally fused to a phenyl ring to form a bicyclic system; wherein the monocyclic heterocyclic ring and the bicyclic system are optionally substituted with one, two, or three substituents independently selected from alkoxy, alkyl, halo, haloalkoxy, and haloalkyl.

The above peptide compounds are further described below.

The present disclosure provides peptide compounds that can inhibit the functioning of the NS3 protease, e.g., in combination with the NS4A protease. Further, the present disclosure describes the administration of combination therapy to a patient whereby a compound in accordance with the present disclosure, which is effective to inhibit the HCV NS3 protease, can be administered with one or two additional compounds having anti-HCV activity.

In a first embodiment the present disclosure provides a compound of Formula (I), or a pharmaceutically acceptable salt thereof, wherein $R^2$ is selected from alkyl and cycloalkyl, wherein the cycloalkyl is optionally substituted with one substituent selected from alkenyl, alkoxy, alkyl, and halo.

In a second embodiment of the first aspect the present disclosure provides a compound of Formula (I), or a pharmaceutically acceptable salt thereof, wherein $R^3$ and $R^4$ are each hydrogen and Q is a $C_6$ unsaturated chain containing zero heteroatoms.

In a third embodiment of the first aspect the present disclosure provides a compound of Formula (I), or a pharmaceutically acceptable salt thereof, wherein

Q is a $C_6$ unsaturated chain containing zero heteroatoms;

$R^1$ is isoquinolinyl optionally substituted with one or two $R^7$ groups;

$R^2$ is selected from alkyl and cycloalkyl, wherein the cycloalkyl is optionally substituted with one substituent selected from alkenyl, alkoxy, alkyl, and halo;

$R^3$ and $R^4$ are each hydrogen; and

$R^6$ is a six-membered fully unsaturated ring containing one nitrogen atom wherein the ring is optionally substituted with one, two, three, or four substitutents independently selected from alkoxy, alkoxycarbonyl, alkyl, alkylcarbonyl, alkylsulfanyl, aryl, carboxy, cyano, cycloalkyl, cycloalkyloxy, halo, haloalkyl, haloalkoxy, $-NR^cR^d$, $(NR^eR^f)$carbonyl, $(NR^eR^f)$sul-

fonyl, and oxo; provided that all substituents on the ring other than fluoro must be in the meta and/or para positions relative to the ring's point of attachment to the parent molecular moiety.

In a second aspect the present disclosure provides a composition comprising a compound of formula (I), or a pharmaceutically acceptable salt thereof, and a pharmaceutically acceptable carrier. In a first embodiment of the second aspect the composition further comprises at least one additional compound having anti-HCV activity. In a second embodiment of the second aspect at least one of the additional compounds is an interferon or a ribavirin. In a third embodiment of the second aspect the interferon is selected from interferon alpha 2B, pegylated interferon alpha, consensus interferon, interferon alpha 2A, and lymphoblastiod interferon tau.

In a fourth embodiment of the second aspect the present disclosure provides a composition comprising a compound of formula (I), or a pharmaceutically acceptable salt thereof, a pharmaceutically acceptable carrier, and at least one additional compound having anti-HCV activity; wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

In a fifth embodiment of the second aspect the present disclosure provides a composition comprising a compound of formula (I), or a pharmaceutically acceptable salt thereof, a pharmaceutically acceptable carrier, and at least one additional compound having anti-HCV activity; wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

In a third aspect the present disclosure provides a compound of formula (I), or a pharmaceutically acceptable salt thereof, for use in a method of treating an HCV infection in a patient, comprising administering to the patient a therapeutically effective amount of a compound of formula (I), or a pharmaceutically acceptable salt thereof. In a first embodiment of the third aspect the method further comprises administering at least one additional compound having anti-HCV activity prior to, after, or simultaneously with the compound of formula (I), or a pharmaceutically acceptable salt thereof. In a second embodiment of the third aspect at least one of the additional compounds is an interferon or a ribavirin. In a fourth embodiment of the third aspect the interferon is selected from interferon alpha 2B, pegylated interferon alpha, consensus interferon, interferon alpha 2A, and lymphoblastiod interferon tau.

In a fifth embodiment of the third aspect the present disclosure provides a compound of formula (I) for use in a method of treating an HCV infection in a patient, comprising administering to the patient a therapeutically effective amount of a compound of formula (I), or a pharmaceutically acceptable salt thereof, and at least one additional compound having anti-HCV activity prior to, after, or simultaneously with the compound of formula (I), or a pharmaceutically acceptable salt thereof, wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

In a sixth embodiment of the third aspect the present disclosure provides a compound of formula (I) for use in a method of treating an HCV infection in a patient, comprising administering to the patient a therapeutically effective amount of a compound of formula (I), or a pharmaceutically acceptable salt thereof, and at least one additional compound having anti-HCV activity prior to, after, or simultaneously with the compound of formula (I), or a pharmaceutically acceptable salt thereof, wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

In a fourth aspect the present disclosure provides a composition comprising a compound of formula (I), or a pharmaceutically acceptable salt thereof, one, two, three, four, or five additional compounds having anti-HCV activity, and a pharmaceutically acceptable carrier. In a first embodiment of the fourth aspect the compsition comprises three or four additional compounds having anti-HCV activity. In a second embodiment of the fourth aspect the composition comprises one or two additional compounds having anti-HCV activity.

In a fifth aspect the present disclosure provides a compound of formula (I) for use in a method of treating an HCV infection in a patient, comprising administering to the patient a therapeutically effective amount of a compound of formula (I), or a pharmaceutically acceptable salt thereof and one, two, three, four, or five additional compounds having anti-HCV activity prior to, after, or simultaneously with the compound of formula (I), or a pharmaceutically acceptable salt thereof. In a first embodiment of the first aspect the method comprises administering three or four additional compounds having anti-HCV activity. In a second embodiment of the first aspect the method comprises administering one or two additional compounds having anti-HCV activity.

Other aspects of the present disclosure may include suitable combinations of embodiments disclosed herein.

Yet other aspects and embodiments may be found in the description provided herein.

The description of the present disclosure herein should be construed in congruity with the laws and principals of chemical bonding. In some instances it may be necessary to remove a hydrogen atom in order accommodate a

substitutent at any given location.

It should be understood that the compounds encompassed by the present disclosure are those that are suitably stable for use as pharmaceutical agent.

It is intended that the definition of any substituent or variable at a particular location in a molecule be independent of its definitions elsewhere in that molecule.

As used in the present specification, the following terms have the meanings indicated:

As used herein, the singular forms "a", "an", and "the" include plural reference unless the context clearly dictates otherwise.

Unless stated otherwise, all aryl, cycloalkyl, and heterocyclyl groups of the present disclosure may be substituted as described in each of their respective definitions. For example, the aryl part of an arylalkyl group may be substituted as described in the definition of the term 'aryl'.

In some instances, the number of carbon atoms in any particular group is denoted before the recitation of the group. For example, the term "$C_6$ alkyl" denotes an alkyl group containing six carbon atoms. Where these designations exist they supercede all other definitions contained herein.

As used herein, the singular forms "a", "an", and "the" include plural reference unless the context clearly dictates otherwise.

The term "alkenyl," as used herein, refers to a straight or branched chain group of two to six carbon atoms containing at least one carbon-carbon double bond.

The term "alkoxy," as used herein, refers to an alkyl group attached to the parent molecular moiety through an oxygen atom.

The term "alkoxyalkyl," as used herein, refers to an alkyl group substituted with one, two, or three alkoxy groups.

The term "alkoxycarbonyl," as used herein, refers to an alkoxy group attached to the parent molecular moiety through a carbonyl group.

The term "alkyl," as used herein, refers to a group derived from a straight or branched chain saturated hydrocarbon containing from one to ten carbon atoms.

The term "alkylcarbonyl," as used herein, refers to an alkyl group attached to the parent molecular moiety through a carbonyl group.

The term "alkylsulfanyl," as used herein, refers to an alkyl group attached to the parent molecular moiety through a sulfur atom.

The term "alkylsulfonyl," as used herein, refers to an alkyl group attached to the parent molecular moiety through a sulfonyl group.

The term "aminocarbonyl," as used herein, refers to an $-NH_2$ group attached to the parent molecular moiety through a carbonyl group.

The term "aryl," as used herein, refers to a phenyl group, or a bicyclic fused ring system wherein one or both of the rings is a phenyl group. Bicyclic fused ring systems consist of a phenyl group fused to a four- to six-membered aromatic or non-aromatic carbocyclic ring. The aryl groups of the present disclosure can be attached to the parent molecular moiety through any substitutable carbon atom in the group. Representative examples of aryl groups include, but are not limited to, indanyl, indenyl, naphthyl, phenyl, and tetrahydronaphthyl. The aryl groups of the present disclosure can be optionally substituted with one, two, three, four, or five substituents independently selected from alkoxy, alkoxycarbonyl, alkyl, alkylcarbonyl, carboxy, cycloalkyl, cycloalkyloxy, cyano, halo, haloalkoxy, haloalkyl, nitro, $-NR^cR^d$, $(NR^cR^d)$carbonyl, and oxo.

The term "arylalkyl," as used herein, refers to an alkyl group substituted with one, two, or three aryl groups.

The term "arylalkylcarbonyl," as used herein, refers to an arylalkyl group attached to the parent molecular moeity through a carbonyl group.

The term "arylcarbonyl," as used herein, refers to an aryl group attached to the parent molecular moiety through a carbonyl group.

The term "arylsulfonyl," as used herein, refers to an aryl group attached to the parent molecular moiety through a sulfonyl group.

The term "carbonyl," as used herein, refers to -C(O)-.

The term "carboxy," as used herein, refers to $CO_2H$.

The term "cyano," as used herein, refers to -CN.

The term "cyanoalkyl," as used herein, refers to an alkyl group substituted with one, two, or three cyano groups.

The term "cycloalkenyl," as used herein, refers to a non-aromatic, partially unsaturated monocyclic, bicyclic, or tricyclic ring system having three to fourteen carbon atoms and zero heteroatoms. Representative examples of cycloalkenyl groups include, but are not limited to, cyclohexenyl, octahydronaphthalenyl, and norbornylenyl.

The term "cycloalkyl," as used herein, refers to a saturated monocyclic or bicyclic hydrocarbon ring system

having three to ten carbon atoms and zero heteroatoms. Representative examples of cycloalkyl groups include, but are not limited to, cyclopropyl, cyclobutyl, and cyclopentyl.

The term "(cycloalkyl)alkyl," as used herein, refers to an alkyl group substituted with one, two, or three cycloalkyl groups.

The term "cycloalkyloxy," as used herein, refers to a cycloalkyl group attached to the parent molecular moiety through an oxygen atom.

The term "dialkylaminocarbonyl," as used herein, refers to an -NR'R" group attached to the parent molecular moiety through a carbonyl group, wherein R' and R" are the same or different alkyl groups.

The term "dialkylaminocarbonylalkyl," as used herein, refers to an alkyl group substituted with one, two, or three dialkylaminocarbonyl groups.

The terms "halo" and "halogen," as used herein, refer to F, Cl, Br, and I.

The term "haloalkoxy," as used herein, refers to a haloalkyl group attached to the parent molecular moiety through an oxygen atom.

The term "haloalkoxyalkyl," as used herein ,refers to an alkyl group substituted with one, two, or three haloalkoxy groups.

The term "haloalkyl," as used herein, refers to an alkyl group substituted with one, two, three, or four halogen atoms.

The term "heterocyclyl," as used herein, refers to a five-, six-, or seven-membered ring containing one, two, or three heteroatoms independently selected from nitrogen, oxygen, and sulfur. The five-membered ring has zero to two double bonds and the six- and seven-membered rings have zero to three double bonds. The term "heterocyclyl" also includes bicyclic groups in which the heterocyclyl ring is fused to a four- to six-membered aromatic or non-aromatic carbocyclic ring or another monocyclic heterocyclyl group. The heterocyclyl groups of the present disclosure can be attached to the parent molecular moiety through a carbon atom or a nitrogen atom in the group. Examples of heterocyclyl groups include, but are not limited to, benzothienyl, furyl, imidazolyl, indolinyl, indolyl, isothiazolyl, isoxazolyl, morpholinyl, oxazolyl, piperazinyl, piperidinyl, pyrazolyl, pyridinyl, pyrrolidinyl, pyrrolopyridinyl, pyrrolyl, thiazolyl, thienyl, and thiomorpholinyl. The heterocyclyl groups of the present disclosure can be optionally substituted with one, two, three, four, or five substituents independently selected from alkoxy, alkoxycarbonyl, alkyl, alkylcarbonyl, carboxy, cycloalkyl, cycloalkyloxy, cyano, halo, haloalkoxy, haloalkyl, nitro, $-NR^cR^d$, $(NR^cR^d)$carbonyl, and oxo.

The term "heterocyclylalkyl," as used herein, refers to an alkyl group substituted with one, two, or three heterocyclyl groups.

The term "heterocyclylalkylcarbonyl," as used herein, refers to a heterocyclylalkyl group attached to the parent molecular moiety through a carbonyl group.

The term "heterocyclylcarbonyl," as used herein, refers to a heterocyclyl group attached to the parent molecular moiety through a carbonyl group.

The term "hydroxy," as used herein, refers to -OH.

The term "hydroxyalkyl," as used herein, refers to an alkyl group substituted with one, two, or three hydroxy groups.

The term "nitro," as used herein, refers to $-NO_2$.

The term "$-NR^aR^b$" as used herein, refers to two groups, $R^a$ and $R^b$, which are attached to the parent molecular moiety through a nitrogen atom. $R^a$ and $R^b$ are independently selected from hydrogen, alkoxy, alkyl, aryl, arylalkyl, cycloalkyl, (cycloalkyl)alkyl, heterocyclyl, and heterocyclylalkyl; or $R^a$ and $R^b$ together with the nitrogen atom to which they are attached form a five or six-membered monocyclic heterocyclic ring.

The term "$-NR^cR^d$," as used herein, refers to two groups, $R^c$ and $R^d$, which are attached to the parent molecular moiety through a nitrogen atom. $R^c$ and $R^d$ are independently selected from hydrogen, alkoxycarbonyl, alkyl, and alkylcarbonyl.

The term "$(NR^cR^d)$alkoxy," as used herein, refers to an $(NR^cR^d)$alkyl group attached to the parent molecular moiety through an oxygen atom.

The term "$(NR^cR^d)$alkyl," as used herein, refers to an alkyl group substituted with one, two, or three $-NR^cR^d$ groups.

The term $(NR^cR^d)$carbonyl," as used herein, refers to an $NR^cR^d$ group attached to the parent molecular moiety through a carbonyl group.

The term "$-NR^eR^f$," as used herein, refers to two groups, $R^e$ and $R^f$, which are attached to the parent molecular moiety through a nitrogen atom. $R^e$ and $R^f$ are independently selected from hydrogen, alkyl, aryl, and arylalkyl.

The term "$(NR^eR^f)$carbonyl," as used herein, refers to an $-NR^eR^f$ group attached to the parent molecular moiety through a carbonyl group.

The term "$(NR^eR^f)$sulfonyl," as used herein, refers to an $-NR^eR^f$ group attached to the parent molecular moiety through a sulfonyl group.

The term "$(NR^gR^h)$carbonyl," as used herein, refers to an $-NR^gR^h$ group attached to the parent molecular moiety

through a carbonyl group.

The term "-$NR^gR^h$," as used herein, refers to two groups, $R^g$ and $R^h$, which are attached to the parent molecular moiety through a nitrogen atom. $R^g$ and $R^h$ are independently selected from hydrogen, alkyl, aryl, arylalkyl, cycloalkyl, (cycloalkyl)alkyl, heterocyclyl, and heterocyclyl; or $R^g$ and $R^h$ together with the nitrogen atom to which they are attached form a monocyclic heterocyclic ring wherein the monocyclic heterocyclic ring is optionally fused to a phenyl ring to form a bicyclic system; wherein the monocyclic heterocyclic ring and the bicyclic system are optionally substituted with one, two, or three substituents independently selected from alkoxy, alkyl, halo, haloalkoxy, and haloalkyl.

The term "oxo," as used herein, refers to =O.

The term "sulfonyl," as used herein, refers to $-SO_2-$.

The term "Prodrug," as used herein, represents compounds which are rapidly transformed *in vivo* to the parent compounds by hydrolysis in blood. Prodrugs of the present disclosure include esters of hydroxy groups on the parent molecule, esters of carboxy groups on the parent molecule, and amides of the amines on the parent molecule.

The compounds of the present disclosure can exist as pharmaceutically acceptable salts. The term "pharmaceutically acceptable salt," as used herein, represents salts or zwitterionic forms of the compounds of the present disclosure which are water or oil-soluble or dispersible, which are, within the scope of sound medical judgment, suitable for use in contact with the tissues of patients without excessive toxicity, irritation, allergic response, or other problem or complication commensurate with a reasonable benefit/risk ratio, and are effective for their intended use. The salts can be prepared during the final isolation and purification of the compounds or separately by reacting a suitable basic functionality with a suitable acid. Representative acid addition salts include acetate, adipate, alginate, citrate, aspartate, benzoate, benzenesulfonate, bisulfate, butyrate, camphorate, camphorsulfonate; digluconate, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Examples of acids which can be employed to form pharmaceutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting an acidic group with a suitable base such as the hydroxide, carbonate, or bicarbonate of a metal cation or with ammonia or an organic primary, secondary, or tertiary amine. The cations af pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, *N*,*N*-dimethylaniline, *N*-methylpiperidine, *N*-methylmorphaline, dicyclohexylamine, procaine, dibenzylamine, *N*,*N*-dibenzylphenethylamine, and *N,N'*-dibenzylethylenediamine. Other representative organic amines useful for the formation of base addition salts include ethylenediamine, ethanolamine, diethanolamine, piperidine, and piperazine.

As used herein, the term "anti-HCV activity" means the compound is effective to treat the HCV virus.

The term "compounds of the disclosure", and equivalent expressions, are meant to embrace compounds of formula (I), and pharmaceutically acceptable enantiomers, diastereomers, and salts thereof. Similarly, references to intermediates, are meant to embrace their salts where the context so permits.

The term "patient" includes both human and other mammals.

The term "pharmaceutical composition" means a composition comprising a compound of the disclosure in combination with at least one additional pharmaceutical carrier, i.e., adjuvant, excipient or vehicle, such as diluents, preserving agents, fillers, flow regulating agents, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms. Ingredients listed in Remington's Pharmaceutical Sciences, 18th ed., Mack Publishing Company, Easton, PA (1999) for example, maybe used.

The phrase "pharmaceutically acceptable" is employed herein to refer to those compounds, materials, compositions, and/or dosage forms which are, within the scope of sound medical judgment, suitable for use in contact with the tissues of patients without excessive toxicity, irritation, allergic response, or other problem or complication commensurate with a reasonable risk/benefit ratio.

The term "therapeutically effective amount" means the total amount of each active component that is sufficient to show a meaningful patient benefit, e.g., a sustained reduction in viral load. When applied to an individual active ingredient, administered alone, the term refers to that ingredient alone. When applied to a combination, the term refers to combined amounts of the active ingredients that result in the therapeutic effect, whether administered in combination, serially or simultaneously.

The terms "treat" and "treating" refers to: (i) preventing a disease, disorder or condition from occurring in a patient which may be predisposed to the disease, disorder and/or condition but has not yet been diagnosed as having

it; (ii) inhibiting the disease, disorder or condition, i.e., arresting its development; and/or (iii) relieving the disease, disorder or condition, i.e., causing regression of the disease, disorder and/or condition.

Where used in naming compounds of the present disclosure, the designations P1', P1, P2, P2*, P3, and P4, as used herein, map the relative positions of the amino acid residues of a protease inhibitor binding relative to the binding of the natural peptide cleavage substrate. Cleavage occurs in the natural substrate between P1 and P1' where the nonprime positions designate amino acids starting from the C-terminus end of the peptide natural cleavage site extending towards the *N*-terminus; whereas, the prime positions emanate from the *N*-terminus end of the cleavage site designation and extend toward the C-terminus. For example, PI' refers to the first position away from the right hand end of the C-terminus of the cleavage site (i.e. *N*-terminus first position); whereas P1 starts the numbering from the left hand side of the C-terminus cleavage site, P2: second position from the C-terminus, etc.). (see Berger A. & Schechter I., Transactions of the Royal Society London series (1970), B257, 249-264].

The following figure shows the subsite designations for the compounds of the present disclosure.

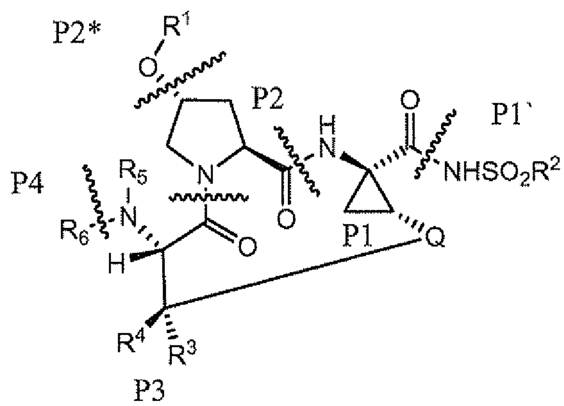

Asymmetric centers exist in the compounds of the present disclosure. For example, the compounds may include P1 cyclopropyl element of formula

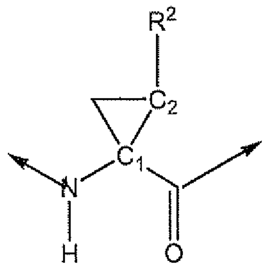

P1

wherein $C_1$ and $C_2$ each represent an asymmetric carbon atom at positions 1 and 2 of the cyclopropyl ring.

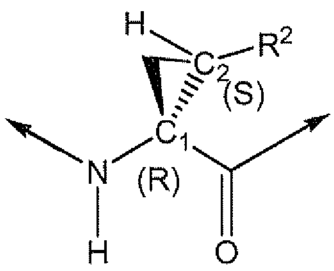

(1R, 2S)
$R^2$ is syn to carbonyl

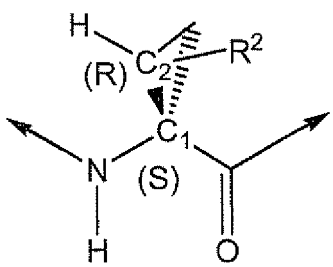

(1S, 2R)
$R^2$ is syn to carbonyl

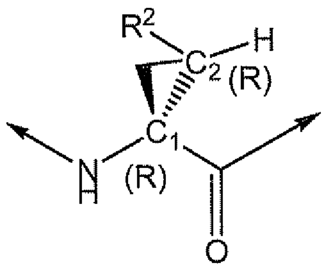

(1R, 2R)
$R^2$ is syn to amide

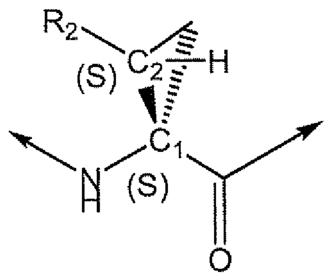

(1S, 2S)
$R^2$ is syn to amide

It should be understood that the disclosure encompasses all stereochemical forms, or mixtures thereof, which possess the ability to inhibit HCV protease.

Certain compounds of the present disclosure may also exist in different stable conformational forms which may be separable. Torsional asymmetry due to restricted rotation about an asymmetric single bond, for example because of steric hindrance or ring strain, may permit separation of different conformers. The present disclosure includes each conformational isomer of these compounds and mixtures thereof.

Certain compounds of the present disclosure may exist in zwitterionic form and the present disclosure includes each zwitterionic form of these compounds and mixtures thereof.

When it is possible that, for use in therapy, therapeutically effective amounts of a compound of formula (I), as well as pharmaceutically acceptable salts thereof, may be administered as the raw chemical, it is possible to present the active ingredient as a pharmaceutical composition. Accordingly, the disclosure further provides pharmaceutical compositions, which include therapeutically effective amounts of compounds of formula (I) or pharmaceutically acceptable salts thereof, and one or more pharmaceutically acceptable carriers, diluents, or excipients. The compounds of formula (I) and pharmaceutically acceptable salts thereof, are as described above. The carrier(s), diluent(s), or excipient(s) must be acceptable in the sense of being compatible with the other ingredients of the formulation and not deleterious to the recipient thereof. In accordance with another aspect of the disclosure there is also provided a process for the preparation of a pharmaceutical formulation including admixing a compound of formula (I), or a pharmaceutically acceptable salt thereof, with one or more pharmaceutically acceptable carriers, diluents, or excipients.

Pharmaceutical formulations may be presented in unit dose forms containing a predetermined amount of active ingredient per unit dose. Dosage levels of between about 0.01 and about 250 milligram per kilogram ("mg/kg") body weight per day, preferably between about 0.05 and about 100 mg/kg body weight per day of the compounds of the disclosure are typical in a monotherapy for the prevention and treatment of HCV mediated disease. Typically, the pharmaceutical compositions of this disclosure will be administered from about 1 to about 5 times per day or alternatively, as a continuous infusion. Such administration can be used as a chronic or acute therapy. The amount of active ingredient that may be combined with the carrier materials to produce a single dosage form will vary depending on the condition being treated, the severity of the condition, the time of administration, the route of administration, the rate of excretion of the compound employed, the duration of treatment, and the age, gender, weight, and condition of the patient. Preferred unit dosage formulations are those containing a daily dose or sub-dose, as herein above recited, or an appropriate fraction thereof, of an active ingredient. Generally, treatment is initiated with small dosages substantially less than the optimum dose of the compound. Thereafter, the dosage is increased by small increments until the optimum effect under the circumstances is reached. In general, the compound is most desirably administered at a concentration level that will generally afford antivirally effective results without causing any harmful or deleterious side effects.

When the compositions of this disclosure comprise a combination of a compound of the disclosure and one or more additional therapeutic or prophylactic agent, both the compound and the additional agent are usually present at dosage levels of between about 10 to 150%, and more preferably between about 10 and 80% of the dosage normally administered in a monotherapy regimen.

Pharmaceutical formulations may be adapted for administration by any appropriate route, for example by the oral (including buccal or sublingual), rectal, nasal, topical (including buccal, sublingual, or transdermal), vaginal, or parenteral (including subcutaneous, intracutaneous, intramuscular, intra-articular, intrasynovial, intrasternal, intrathecal, intralesional, intravenous, or intradermal injections or infusions) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil emulsions.

For instance, for oral administration in the form of a tablet or capsule, the active drug component can be combined with an oral, non-toxic pharmaceutically acceptable inert carrier such as ethanol, glycerol, water, and the like. Powders are prepared by comminuting the compound to a suitable fine size and mixing with a similarly comminuted pharmaceutical carrier such as an edible carbohydrate, as, for example, starch or mannitol. Flavoring, preservative, dispersing, and coloring agent can also be present.

Capsules are made by preparing a powder mixture, as described above, and filling formed gelatin sheaths. Glidants and lubricants such as colloidal silica, talc, magnesium stearate, calcium stearate, or solid polyethylene glycol can be added to the powder mixture before the filling operation. A disintegrating or solubilizing agent such as agar-agar, calcium carbonate, or sodium carbonate can also be added to improve the availability of the medicament when the capsule is ingested.

Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, and the like. Lubricants used in these dosage forms include sodium oleate, sodium chloride, and the like. Disintegrators include, without limitation, starch, methyl cellulose, agar, betonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an aliginate, gelating, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present disclosure can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

Oral fluids such as solution, syrups, and elixirs can be prepared in dosage unit form so that a given quantity contains a predetermined amount of the compound. Syrups can be prepared by dissolving the compound in a suitably flavored aqueous solution, while elixirs are prepared through the use of a non-toxic vehicle. Solubilizers and emulsifiers such as ethoxylated isostearyl alcohols and polyoxyethylene sorbitol ethers, preservatives, flavor additive such as peppermint oil or natural sweeteners, or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax, or the like.

The compounds of formula (I), and pharmaceutically acceptable salts thereof, can also be administered in the form of liposome delivery systems, such as small unilamellar vesicles, large unilamellar vesicles, and multilamellar vesicles. Liposomes can be formed from a variety of phopholipids, such as cholesterol, stearylamine, or phophatidylcholines.

The compounds of formula (I) and pharmaceutically acceptable salts thereof may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols, or oils.

For treatments of the eye or other external tissues, for example mouth and skin, the formulations are preferably applied as a topical ointment or cream. When formulated in an ointment, the active ingredient may be employed with

either a paraffinic or a water-miscible ointment base. Alternatively, the active ingredient may be formulated in a cream with an oil-in-water cream base or a water-in oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles, and mouth washes.

Pharmaceutical formulations adapted for rectal administration may be presented as suppositories or as enemas.

Pharmaceutical formulations adapted for nasal administration wherein the carrier is a solid include a course powder having a particle size for example in the range 20 to 500 microns which is administered in the manner in which snuff is taken, i.e., by rapid inhalation through the nasal passage from a container of the powder held close up to the nose. Suitable formulations wherein the carrier is a liquid, for administration as a nasal spray or nasal drops, include aqueous or oil solutions of the active ingredient.

Pharmaceutical formulations adapted for administration by inhalation include fine particle dusts or mists, which may be generated by means of various types of metered, dose pressurized aerosols, nebulizers, or insufflators.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti-oxidants, buffers, bacteriostats, and soutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets.

It should be understood that in addition to the ingredients particularly mentioned above, the formulations may include other agents conventional in the art having regard to the type of formulation in question, for example those suitable for oral administration may include flavoring agents.

Table 1 below lists some illustrative examples of compounds that can be administered with the compounds of this disclosure. The compounds of the disclosure can be administered with other anti-HCV activity compounds in combination therapy, either jointly or separately, or by combining the compounds into a composition.

*Table 1*

| *Brand Name* | *Physiological Class* | *Type of Inhibitor or Target* | *Source Company* |
|---|---|---|---|
| NIM811 | | Cyclophilin Inhibitor | Novartis |
| Zadaxin | | Immuno-modulator | Sciclone |
| Suvus | | Methylene blue | Bioenvision |
| Actilon (CPG10101) | | TLR9 agonist | Coley |
| Batabulin (T67) | Anticancer | β-tubulin inhibitor | Tularik Inc., South San Francisco, CA |
| ISIS 14803 | Antiviral | antisense | ISIS Pharmaceuticals Inc, Carlsbad, CA/Elan Pharmaceuticals Inc., New York, NY |
| Summetrel | Antiviral | antiviral | Endo Pharmaceuticals Holdings Inc., Chadds Ford, PA |
| GS-9132 (ACH-806) | Antiviral | HCV Inhibitor | Achillion / Gilead |
| Pyrazolopyrimidine compounds and salts From WO-2005047288 26 May 2005 | Antiviral | HCV Inhibitors | Arrow Therapeutics Ltd. |
| Levovirin | Antiviral | IMPDH inhibitor | Ribapharm Inc., Costa Mesa, CA |

(continued)

| *Brand Name* | *Physiological Class* | *Type of Inhibitor or Target* | *Source Company* |
|---|---|---|---|
| Merimepodib (VX-497) | Antiviral | IMPDH inhibitor | Vertex Pharmaceuticals Inc., Cambridge, MA |
| XTL-6865 (XTL-002) | Antiviral | monoclonal antibody | XTL Biopharmaceuticals Ltd., Rehovot, Isreal |
| Telaprevir (VX-950, LY-570310) | Antiviral | NS3 serine protease inhibitor | Vertex Pharmaceuticals Inc., Cambridge, MA/ Eli Lilly and Co. Inc., Indianapolis, IN |
| HCV-796 | Antiviral | NS5B Replicase Inhibitor | Wyeth / Viropharma |
| NM-283 | Antiviral | NS5B Replicase Inhibitor | Idenix / Novartis |
| GL-59728 | Antiviral | NS5B Replicase Inhibitor | Gene Labs / Novartis |
| GL-60667 | Antiviral | NS5B Replicase Inhibitor | Gene Labs / Novartis |
| 2'C MeA | Antiviral | NS5B Replicase Inhibitor | Gilead |
| PSI 6130 | Antiviral | NS5B Replicase Inhibitor | Roche |
| R1626 | Antiviral | NS5B Replicase Inhibitor | Roche |
| 2'C Methyl adenosine | Antiviral | NS5B Replicase Inhibitor | Merck |
| JTK-003 | Antiviral | RdRp inhibitor | Japan Tobacco Inc., Tokyo, Japan |
| Levovirin | Antiviral | ribavirin | ICN Pharmaceuticals, Costa Mesa, CA |
| Ribavirin | Antiviral | ribavirin | Schering-Plough Corporation, Kenilworth, NJ |
| Viramidine | Antiviral | Ribavirin Prodrug | Ribapharm Inc., Costa Mesa, CA |
| Heptazyme | Antiviral | ribozyme | Ribozyme Pharmaceuticals Inc., Boulder, CO |
| BILN-2061 | Antiviral | serine protease inhibitor | Boehringer Ingelheim Pharma KG, Ingelheim, Germany |
| SCH 503034 | Antiviral | serine protease inhibitor | Schering Plough |
| Zadazim | Immune modulator | Immune modulator | SciClone Pharmaceuticals Inc., San Mateo, CA |
| Ceplene | Immunomodulator | immune modulator | Maxim Pharmaceuticals Inc., San Diego, CA |
| CellCept | Immunosuppressant | HCV IgG immunosuppressant | F. Hoffmann-La Roche LTD, Basel, Switzerland |

(continued)

| *Brand Name* | *Physiological Class* | *Type of Inhibitor or Target* | *Source Company* |
|---|---|---|---|
| Civacir | Immunosuppressant | HCV IgG immuno-suppressant | Nabi Biopharmaceutic als Inc., Boca Raton, FL |
| Albuferon - α | Interferon | albumin IFN-α2b | Human Genome Sciences Inc., Rockville, MD |
| Infergen A | Interferon | IFN aliacon-1 | InterMune Pharmaceuticals Inc., Brisbane, CA |
| Omega IFN | Interferon | IFN-ω | Intarcia Therapeutics |
| IFN-β and EMZ701 | Interferon | IFN-β and EMZ701 | Transition Therapeutics Inc., Ontario, Canada |
| Rebif | Interferon | IFN-β1a | Serono, Geneva, Switzerland |
| Roferon A | Interferon | IFN-α2a | F. Hoffmann-La Roche LTD, Basel, Switzerland |
| Intron A | Interferon | IFN-α2b | Schering-Plough Corporation, Kenilworth, NJ |
| Intron A and Zadaxin | Interferon | IFN-α2b/α1-thymosin | RegeneRx Biopharma. Inc., Bethesda, MD/ SciClone Pharmaceuticals Inc, San Mateo, CA |
| Rebetron | Interferon | IFN-α2b/ribavirin | Schering-Plough Corporation, Kenilworth, NJ |
| Actimmune | Interferon | INF-γ | InterMune Inc., Brisbane, CA |
| Interferon-β | Interferon | Interferon-β-1a | Serono, |
| Multiferon | Interferon | Long lasting IFN | Viragen/ Valentis |
| Wellferon | Interferon | Lympho-blastoid IFN-αn1 | GlaxoSmithKline plc, Uxbridge, UK |
| Omniferon | Interferon | natural ICN-α | Viragen Inc., Plantation, FL |
| Pegasys | Interferon | PEGylated IFN-α2a | F. Hoffmann-La Roche LTD, Basel, Switzerland |
| Pegasys and Ceplene | Interferon | PEGylated IFN-α2a/ immune modulator | Maxim Pharmaceuticals Inc., San Diego, CA |
| Pegasys and Ribavirin | Interferon | PEGylated IFN-α2a/ribavirin | F. Hoffmann-La Roche LTD, Basel, Switzerland |
| PEG-Intron | Interferon | PEGylated IFN-α2b | Schering-Plough Corporation, Kenilrworth, NJ |
| PEG-Intron / Ribavirin | Interferon | PEGylated IFN-α2b/ribavirin | Schering-Plough Corporation, Kenilworth, NJ |
| IP-501 | Liver protection | antifibrotic | Indevus Pharmaceuticals Inc., Lexington, MA |
| IDN-6556 | Liver protection | caspase inhibitor | Idun Pharmaceuticals Inc., San Diego, CA |
| ITMN-191 (R-7227) | Antiviral | serine protease inhibitor | InterMune Pharmaceuticals Inc., Brisbane, CA |

(continued)

| Brand Name | Physiological Class | Type of Inhibitor or Target | Source Company |
|---|---|---|---|
| GL-59728 | Antiviral | NS5B Replicase Inhibitor | Genelabs |
| ANA-971 | Antiviral | TLR-7 agonist | Anadys |
| Boceprevir | Antiviral | serine protease inhibitor | Schering Plough |
| TMS-435 | Antiviral | serine protease inhibitor | Tibotec BVBA, Mechelen, Belgium |
| BI-201335 | Antiviral | serine protease inhibitor | Boehringer Ingelheim Pharma KG, Ingelheim, Germany |
| MK-7009 | Antiviral | serine protease inhibitor | Merck |
| PF-00868554 | Antiviral | replicase inhibitor | Pfizer |
| ANA598 | Antiviral | Non-Nucleoside NS5B Polymerase Inhibitor | Anadys Pharmaceuticals, Inc., San Diego, CA, USA |
| IDX375 | Antiviral | Non-Nucleoside Replicase Inhibitor | Idenix Pharmaceuticals, Cambridge, MA, USA |
| BILB 1941 | Antiviral | NS5B Polymerase Inhibitor | Boehringer Ingelheim Canada Ltd R&D, Laval, QC, Canada |
| PSI-7851 | Antiviral | Nucleoside Polymerase inhibitor | Pharmasset, Princeton, NJ, USA |
| VCH-759 | Antiviral | NS5B Polymerase Inhibitor | ViroChem Pharma |
| VCH-916 | Antiviral | NS5B Polymerase Inhibitor | ViroChem Pharma |
| GS-9190 | Antiviral | NS5B Polymerase Inhibitor | Gilead |
| Peg-interferon lamda | Antiviral | Interferon | ZymoGenetics/B ristol-Myers Squibb |

The compounds of the disclosure may also be used as laboratory reagents. Compounds may be instrumental in providing research tools for designing of viral replication assays, validation of animal assay systems and structural biology studies to further enhance knowledge of the HCV disease mechanisms. Further, the compounds of the present disclosure are useful in establishing or determining the binding site of other antiviral compounds, for example, by competitive inhibition.

The compounds of this disclosure may also be used to treat or prevent viral contamination of materials and therefore reduce the risk of viral infection of laboratory or medical personnel or patients who come in contact with such materials, e.g., blood, tissue, surgical instruments and garments, laboratory instruments and garments, and blood collection or transfusion apparatuses and materials.

This disclosure is intended to encompass compounds having formula (I) when prepared by synthetic processes or by metabolic processes including those occurring in the human or animal body (*in vivo*) or processes occurring *in nitro*,

The abbreviations used in the present application, including particularly in the illustrative schemes and examples which follow, are well-known to those skilled in the art. Some of the abbreviations used are as follows: EtOAc for ethyl acetate; t-Bu for tert-butyl; DMSO for dimethylsulfoxide; HATU for *O*-(7-azabenzotriazol-1-yl)-*N*,*N*,*N*',*N*'-tetramethyluronium phosphate; DIEA or DIPEA for diisopropylethylamine; DCM for dichloromethane; CDI for 1,1'-carbonyldiimidazole; DBU for 1,8-diazabicyclo[5.4.0]undec-7-ene; h for hours; min for minutes; MeOH for methanol; NBS for N-bramosuccinimide; n-Bu-Li for n-butyllithium; i-Pr for isopropyl; TMS for trimethylsilyl; THF for tetrahydrofuran; MeCN for acetonitrile; and rt for room temperature or retention time (context will dictate).

The starting materials useful to synthesize the compounds of the present disclosure are known to those skilled in the art and can be readily manufactured or are commercially available.

The following methods set forth below are provided for illustrative purposes and are not intended to limit the scope of the claims. It will be recognized that it may be necessary to prepare such a compound in which a functional group is protected using a conventional protecting group then to remove the protecting group to provide a compound of the present disclosure. The details concerning the use of protecting groups in accordance with the present disclosure are known to those skilled in the art.

*Example 1: Preparation of Compound 1*

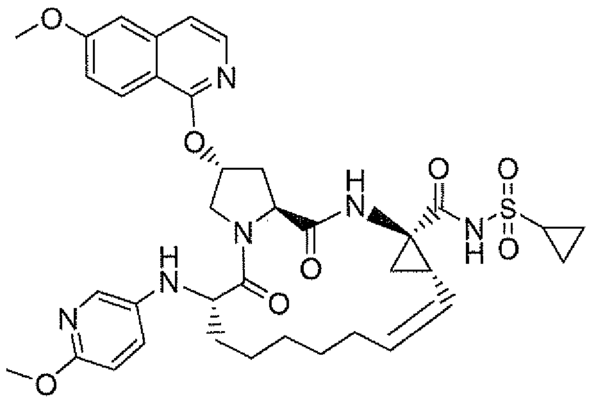

Compound 1

Scheme 1

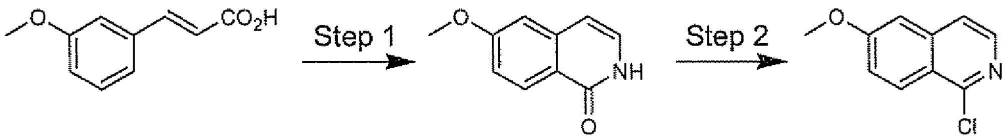


*Step 1:*

To a solution of 3-methoxycinnamic acid (11.0 g, 62 mmol) and triethylamine (12.5 g, 124 mmol) in acetone (80 mL) was added ethyl chloroformate (approximately 1.5 equivalents) dropwise at 0 °C. After stirring at this temperature for 1 hour, aqueous $NaN_3$ (6.40 g, 100 mmol in 35 mL $H_2O$) was added dropwise and the reaction mixture was stirred for 16 hours at ambient temperature. Water (100 mL) was added to the mixture and volatiles were removed *in vacuo.* The resulting slurry was extracted with toluene (3 x 50 mL) and the organic layers were combined, dried over $MgSO_4$ and filtered. The filtrate was added dropwise to a heated solution of diphenylmethane (50 mL) and tributylamine (30 mL) at 190 °C. The toluene was removed by distillation during the addition. After complete addition, the reaction temperature was raised to 210 °C for 2 hours. Upon cooling, the precipitated product was collected by filtration, washed with hexane (2 x 50 mL), and dried to provide the desired product as a white solid (5.53 g, 51%) (Nicolas Briet et al, Tetrahedron, 2002, 5761-5766). LC-MS, MS *m/z* 176 ($M^+$+H).

*Step 2:*

6-Methoxy-2*H*-isoquinolin-1-one (5.0 g, 28.4 mmol) in $POCl_3$ (10 mL) was heated to gentle reflux for 3 hours and the mixture was then concentrated *in vacuo* (Nicolas Briet et al, Tetrahedron, 2002, 5761-5766). The residue was poured into ice water (20 mL) and brought to pH = 10 by addition of 10.0M NaOH. The resulting mixture was extracted with $CHCl_3$. The organic layer was washed with brine, dried over $MgSO_4$ filtered and concentrated. The residue was

purified by flash chromatography (1:1 hexane-ethyl acetate) to provide 4.41 g (80%) of the desired product as a white solid. $^1H$ NMR ($CD_3OD$) δ ppm 3.98 (s, 3H), 7.34-7.38 (m, 2H), 7.69 (d, *J* = 5.5Hz, 1H), 8.10 (d, *J* = 6.0 Hz, 1H), 8.23 *(d, J*= 9.5 Hz, 1H); LC-MS, MS m/z 194 ($M^+$+H).

Scheme 2

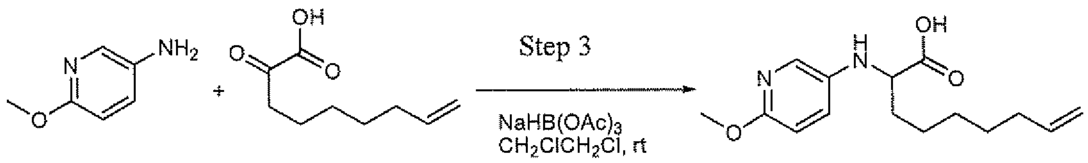


*Step 3:*

A mixture of 6-methoxypyridin-3-amine (0.372 g, 3 mmol), 2-oxonon-8-enoic acid (0.511 g, 3.00 mmol), and $NaHB(OAc)_3$ (1.907 g, 9.00 mmol) in $CH_2ClCH_2Cl$ (10 ml) was stirred for 24 h. The reaction was quenched with saturated $NH_4Cl$ (20 mL), diluted with EtOAc (50 mL), washed with brine (20 ml), dried over $MgSO_4$, filtrated, concentrated. Concentration gave 800 mg of a crude desired product which was used in the next step without further purification.

Scheme 3

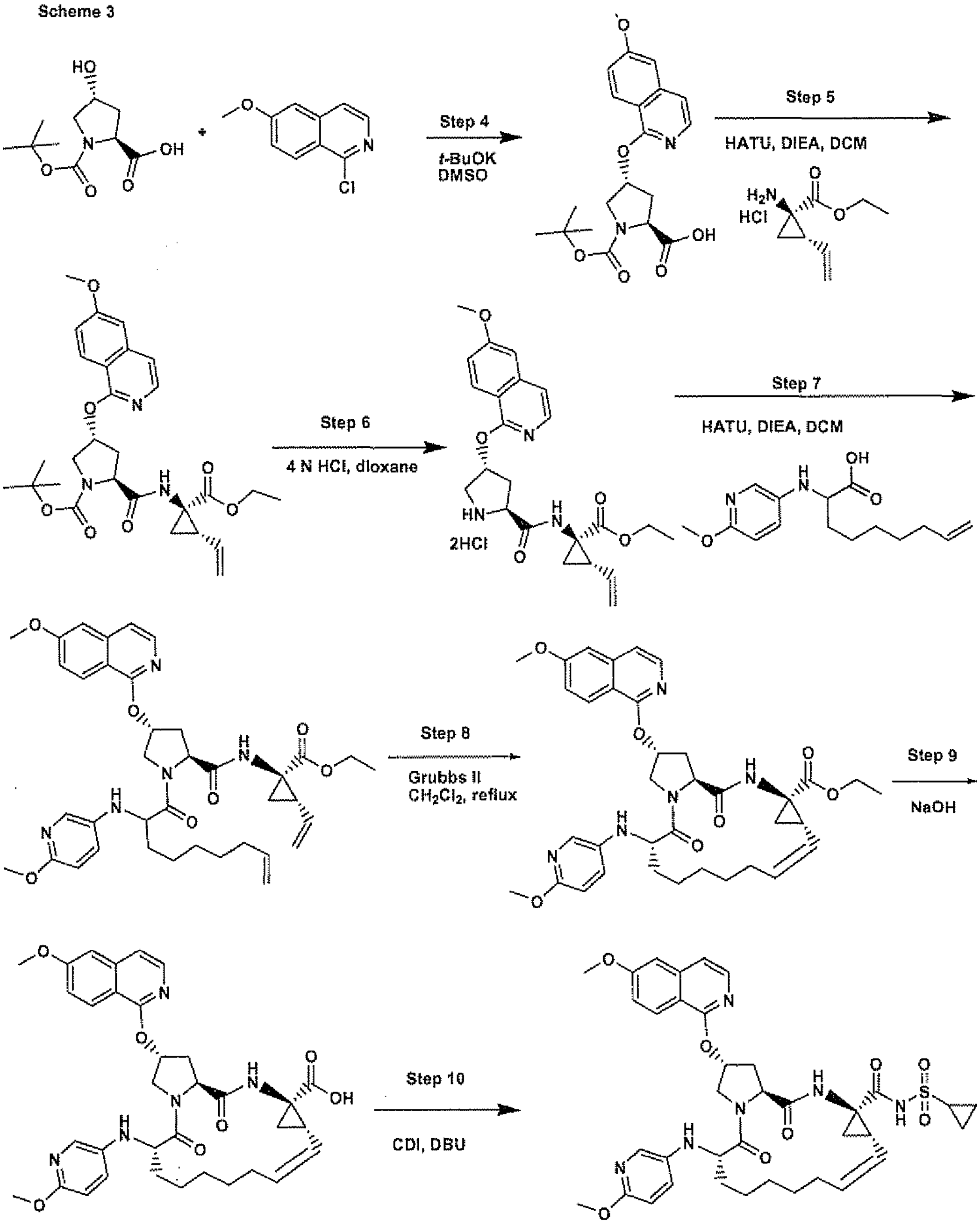


*Step 4:*

To a solution of *N*-Boc-4-(*R*)-hydroxy-L-proline (0.892 g, 3.89 mmol) in DMSO (40 mL) at ambient temperature was added solid potassium *tert*-butoxide (1.34 g, 12.0 mmol) in one portion. The suspension was stirred at room temperature for 30 minutes before being cooled to 10°C. 1-Chloro-6-methoxy-isoquinoline (the product of Step 2, Example 1) (785 mg, 4.05 mmol) was added as a solid in one portion and the resulting mixture was stirred at ambient temperature for 12 hours. The mixture was quenched with ice cold 5% citric acid (aq) and was then extracted with ethyl acetate (100 mL). The aqueous phase was extracted with ethyl acetate once more. The combined organic layers were washed with 5% citric acid (aq) and brine respectively, dried over $MgSO_4$ and filtered. The filtrate was concentrated *in vacuo* to dryness to provide the desired product as an off-white foam (1.49 g, 99% yield). This crude material was used in the next reaction step without further purification. $^1$H NMR ($CD_3OD$) δ 1.42, 1.44 (rotamers, 9H), 2.38-2.43 (m, 1H), 2.66-2.72 (m, 1H), 3.80-3.87 (m, 2H), 3.92 (s, 3H), 4.44-4.52 (m, 1H), 5.73 (bs, 1H), 7.16-7.18 (m, 2H), 7.24-7.25 (m, 1H), 7.87-7.88 (m, 1H), 8.07 (d, *J* = 8.5 Hz, 1H); LC-MS, MS *m/z* 389 ($M^+$+H).

*Step 5:*

To a mixture of the product of Step 4, Example 1 (7.7 g, 20 mmol), DIPEA (12.92 g, 100 mmol), and (1R,2S)-1-amino-N-(cyclopropylsulfonyl)-2-vinylcyclopropanecarboxamide HCl salt (4.6 g, 24 mmol) in $CH_2Cl_2$ (100 mL) was added HATU (11.41 g, 30 mmol) at rt for 4 hours. After concentration, the residue was purified by Biotage (40+S Si column) eluting with 33% acetone in hexanes to give 9.5 g (90%) of the desired product as an oil. LC-MS, MS *mlz* 526 ($M^+$+H).

*Step 6:*

To the product of Step 5, Example (5.26 g, 10 mmol) was added 4M HCl (25.00 mL, 100 mmol) in 1,4-dioxane. The formed solution was stirred at 25 °C for 3 h. After concentration under vacuo, to the residue was added ether (20mL), then concentrated again, repeated the procedure 3 times. House vacuum drying gave 4.98 g (100%) of the crude product as a solid, which was used in the next step without further purification. LC-MS, MS *mlz* 426 ($M^+$+H).

*Step 7:*

To a solution of the product of Step 6, Example 1 (150 mg, 0.3mmol), the product of Step 3, Example 1 (84 mg, 0.300 mmol), and Hunig'sBase (0.524 ml, 3.00 mmol) in $CH_2Cl_2$ (5 ml) was added HATU (171 mg, 0.450 mmol). After stirring for 16 h and concentration, the residue was purified by Biotage eluting with 33% acetone in hexanes to give 170 mg of the desired products as a solid and diastereomer. LC-MS, MS *m*/*z* 686 ($M^+$+H).

*Step 8:*

A solution of the product of Step 7, Example 1 (160 mg, 0.233 mmol) and GrubbsII (30 mg, 0.035 mmol) in $CH_2Cl_2$ (5 ml) was added refluxed for 16 h. After concentration, the residue was purified by Biotage eluting with 25% acetone in hexanes to give 52 mg of a single diastereomer whose structure was labeled either as shown in Scheme or (*R*)-isomer. LC-MS, MS m/z 658 ($M^+$+H).

Step 9:

A solution of the product of Step 8, Example 1 (50 mg, 0.076 mmol) and sodium hydroxide (30.4 mg, 0.760 mmol) in MeOH (5 ml) and Water (2.00 ml) was refluxed for 2 h. After concentration, the residue was neutralized with 1 N HCl (1 mL), extracted with EtOAc, dried over $MgSO_4$, Concentration gave 36 mg of the desired product which was used in the next step without further purification. LC-MS, MS *m*/*z* 630 ($M^+$+H).

*Step 10:*

A solution of the product of Step 9, Example 1 (35 mg, 0.056 mmol) and CDI (12.62 mg, 0.078 mmol) in THF (1 ml) was refluxed for 1 h. After cooling to rt, to the solution was added cyclopropanesulfonamide (10.77 mg, 0.089 mmol) followed by DBU (0.017 ml, 0.111 mmol), then the resulting solution was stirred overnight. After concentration, the residue was purified by prep HPLC to give 25 mg (61%) of the desired product as a white solid. LC-MS, MS m/z 733 ($M^+$+H).

*Example 2: Reparation of Compound 2*

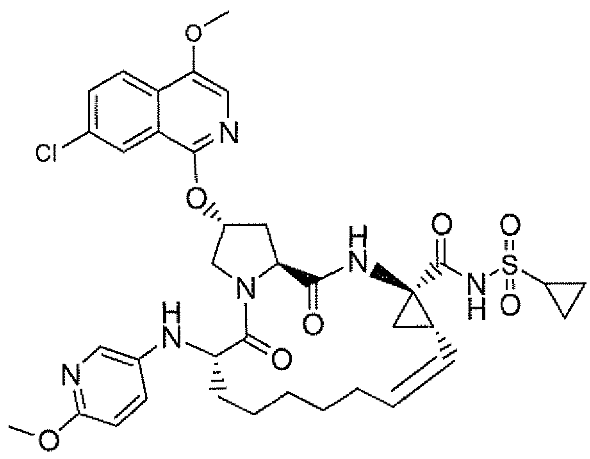

Compound 2

Scheme 1

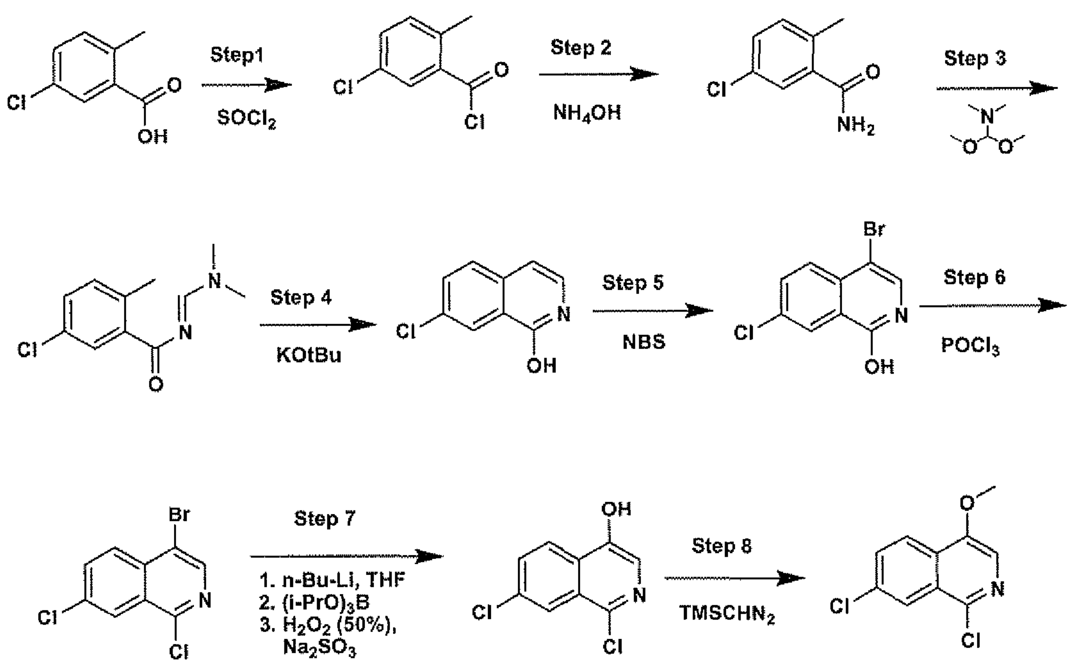


*Step 1:*

A slurry of 3-chloro-6-methylbezoic acid (17.0 g, 0.10 mol) in thionyl chloride (23.5 ml, 0.30 mol) was heated slowly to a gentle reflux and maintained at this temperature for 2h. The reaction mixture was then cooled to RT and the excess thionyl chloride removed in vacuo. The residue was taken up in DCM (50 ml), and the solvent then removed *in vacuo*. (It should be noted that this process was repeated several times to ensure removal of residual thionyl chloride and HCl). The resulting product was then dissolved in THF (80 ml) which was used directly in the next reaction as described below.

*Step 2:*

To a solution of 30% ammonia (58 ml) in water (240 ml), cooled by salt-ice bath (-10 °C), was added dropwise a THF solution of the product of Step 1, Example 2. After the addition was complete, the resulting reaction mixture (slurry) was stirred at -10 °C for 1 hr. The reaction mixture was then warmed to room temperature and decanted. The remaining solid in the reaction vessel was then triturated with water (50 ml). This process of trituration and decanting

was then repeated. The remaining solid was then filtered and the filter cake washed with water. The solid was then dried *in vacuo* overnight to yield 13.8 g (82%) of desired product as a white crystalline material. $^1H$ NMR (DMSO-$d_6$) δ ppm 2.33 (s, 3H), 7.24-7.27 (m, 1H), 7.35-7.38 (m , 2H), 7.44 (b, 1H), 7.80 (b, 1H); $^{13}C$ NMR (100 MHz, DMSO-$d_6$) δ ppm 18.87, 126.64, 128.86, 129.81, 132.31, 134.19, 138.65, 169.41; LC-MS, MS *m*/*z* 170.

*Step 3:*

A mixture of the product of Step 2, Example 2 (11.5 g, 68 mmol), DMF-acetal (10.9 ml, 82 mmol), and THF (150 ml) was heated to reflux and maintained at this temperature for 2 hr. The reaction mixture was then cooled to room temperature and the volatiles were removed in vacuo. The resulting residue was recrystallized from hexane (150 ml) to yield 14.7 g (96%) of the desired product as white needles. $^1H$ NMR (DMSO-$d_6$) δ 2.49-2.51 (m, 3H), 3.09 (s, 3H), 3.20 (s, 3H), 7.24, 7.27 (d, J=13.5 Hz, 1H), 7.37-7.41 (dd, J1=14 Hz, J2=4.5 Hz, 1H), 7.91, 7.92 (d, J=4.0 Hz, 1H), 8.55 (s, 1H); $^{13}C$ NMR (100 MHz, DMSO-$d_6$) δ ppm 20.69,35.09,40.91, 129.50, 129.72, 132.98, 136.86,138.87, 160.60, 177.04; LC-MS, MS *m*/*z* 225.

) *Step 4:*

A mixture of the product of Step 3, Example 2 and KOtBu (14.7 g, 131 mmol) in THF (300 ml) was heated to reflux and maintained at this temperature for 2 hr (reaction mixture became a dark solution upon heating). The volume of the reaction mixture was then reduced by distilling off approximately 100 ml of solvent. The resulting solution was then carefully poured into water ( 1 L) and the resulting mixture was acidified with 1M HCl to a resulting pH of 4. The mixture was then filtered, and the collected solid was washed thoroughly with water, then dried in vacuo overnight to yield 7.0 g (60%) of the desired product as a off-white powder. $^1H$ NMR (400 Hz, $CD_3OD$) δ ppm 6.66 (d, J=7.05 Hz, 1 H), 7.18 (d, J=7.05 Hz, 1 H), 7.66 (s, 1 H) 7.67 (d, J=2.01 Hz, 1 H), 8.24 (d, J=2.27 Hz, 1 H); $^{13}C$ NMR (100 MHz, DMSO-$d_6$) δ ppm 104.05, 125.62, 127.21, 128.54, 129.52, 130.77, 132.43, 136.55, 160.72; LC-MS, MS *m*/*z* 180.

*Step 5:*

A slurry of the product of Step 4, Example 2 and NBS (39.747 g, 223.3 mmol) in MeCN (500 mL, anhydrous) was slowly heated to a gentle reflux over a period of approximately 2 h and maintained at a gentle reflux for 1.5 h. (This reaction can be monitored by LC/MS). The reaction mixture was then slowly cooled to room temperature over a period of 3 h and the observed solid was removed by simple filtration. The collected solid was washed with MeCN (100 mL x 3) to provide 47 g of the desired product. This material was used in the next step without further purification. $^1H$ NMR (400 MHz, $CD_3OD$) δ ppm 7.46(s, 1H), 7.81 (dd, J=8.40, 2.00 Hz, 1 H), 7.88 (d, J=8.8 Hz, 1 H), 8.27(d, J=2.00 Hz, 1 H); $^{13}C$ NMR (100 MHz, DMSO-$d_6$) δ ppm 96,68, 126.34, 127.58, 127.71, 130.73, 132.20, 133.47, 134.46,159.88; LC-MS, MS *m*/*z* 258.

*Step 6:*

A heterogeneous solution of the product of Step 5, Example 2 (47 g, 182 mmol) in $POCl_3$ (200 mL, 2.15 mol) slowly heated to reflux over a period of 1 h. The reaction mixture was maintained at reflux for 4 h. The reaction mixture was then cooled to room temperature and concentrated in vacuo to remove excess $POCl_3$. The resulting residue was then taken-up into 600 mL of $CH_2Cl_2$, cooled to at -35 °C, then neutralized carefully with 1 N NaOH (400 mL) until the mixture was slightly basic (pH = 8). The resulting organic layer was separated, washed with water, dried over $MgSO_4$ and concentrated in vacuo. The resulting residue was crystallized from EtOAc (approximately 50 mL) to give 32 g of desired product. The collected solid was washed with 10% EtOAc/Hexanes (3 x 50 ml). The mother liquid was concentrated and purified by Biotage (elution with 16% EtOAc in hexanes) to give 4 g of the desired product as a solid, $^1H$ NMR (400 MHz, $CDCl_3$) δ ppm 7.8ml (dd, J=8.81, 2.01 Hz, 1 H), 8.14 (d, J=9.06 Hz, 1 H), 8.34 (d, J=1.76 Hz, 1 H), 8.48 (s, 1 H); $^{13}C$ NMR (100 MHz, DMSO-$d_6$) δ ppm 118.39, 125.06, 127.59, 128.71, 133.89, 134.14, 134.93, 143.18, 148.98; LC-MS, MS *m*/*z* 275.

*Step 7:*

To a slurry of the product of Step 6, Example 2 (22.16 g, 80 mmol) in THF (500 ml) at -78 °C was added 100 ml of 1.6 M n-BuLi (in hexanes, 160 mmol) dropwise via cannula over 15 min (maintaining the internal temperature < -65 °C). The resulting solution was stirred for 0.5 h, after such time, $(i\text{-}PrO)_3B$ (37 ml, 160 mmol) was added dropwise via syringe over 10 min (maintaining the internal temperature < -65 °C). The resulting reaction mixture was stirred for 0.5 h. After checking the reaction by LC/MS for completion, 80 ml of 30% $H_2O_2$ (776 mmol) was added dropwise via

addition funnel over 10 min (the internal temperature rose to -60 °C during addition), followed by addition of 80 ml of 1 N NaOH (80 mmol). The cooling bath was removed, and the reaction mixture was allowed to warm to room temperature and stirred at room temperature for additional 1 h. After conforming the completion of the reaction by LC/MS, the reaction mixture was then cooled to -40 C, and a solution of 100 g of $Na_2SO_3$ (0.793 moles) in 400 ml of water was added dropwise via addition funnel as a means to quench excess $H_2O_2$ over 30 min (maintaining the internal temperature 5-10 °C). The resulting slurry was then neutralized with 6 N HCl (approximately 50 ml) at 0 °C till pH ~ 6, then diluted with 500 ml of EtOAc and decanted to a 2 L separatory funnel. To the remaining solid in the reaction vessel was added 500 mL of water and 300 ml of EtOAc, then neutralized with 6 N HCl (approximately 20 ml). The combined organic layers were washed with brine (300 ml x 3), then water (200 ml x 3), dried over $MgSO_4$ and concentrated to give a crude product which was triturated with 50 ml of EtOAc. The solid was collected by filtration, rinsed with EtOAc (3 x 25 ml) and dried to give desired product (2 runs: 12.0 g, 70% and 13.8 g, 81 %). The filtrates were combined, concentrated and purified by Biotage eluted with 35% EtOAc in hexanes to give 2.1 g of product. Overall, 44.4 g of bromide gave 27.9 g (81 %) of 4-OH product. $^1$H NMR (400 MHz, $CD_3OD$) δ ppm 4.05 (s, 3 H), 7.4 (s, 1 H), 7.76 (dd, J=8.8, 2, Hz, 1 H), 8.16 (d, J=2 Hz, 1 H), 8.23 (d, J=8.8 Hz, 1 H); $^{13}$C NMR (100 MHz, DMSO-$d_6$) δ ppm 123.78, 124.66, 125.62, 127.03, 127.71, 130.72, 133.80, 137.63; 148.88; LC-MS, MS *m*/*z* 213.

*Step 8:*

To a slurry of the product of Step 7, Example 2 (16 g, 75.5 mmol) in MeOH-MeCN (30 mL/300 mL) at 0 0C was added dropwise 60 ml of 2 M solution of $TMSCHN_2$ in hexanes (120 mmol). The reaction mixture was allowed to warm to room temperature, then stirred for 14 h. The solution was then concentrated and the resulting solid was recrystallized from EtOAc (about 50 mL) to give 8.1 g of the desired product which was washed with 25% EtOAc in hexances; 20 x3 times. The mother liquid was concentrated and purified by Biotage (elution with 16 % EtOAc in hexanes) to provide 3.2 g of the desired product as a solid. $^1$H NMR (400 MHz, $CDCl_3$) δ ppm 4.05 (s, 3 H), 7.67 (dd, J=9.06, 2.01 Hz, 1 H), 7.80 (s, 1 H), 8.16 (d, J=8.81 Hz, 1 H), 8.23 (d, J=2.01 Hz, 1 H); $^{13}$C NMR (100 MHz, DMSO-$d_6$) δ ppm 56.68, 122.70, 123.99, 124.14, 126.67, 127.83, 131.43, 134.10, 139.75, 149.94; LC-MS, MS *m*/*z* 229.

Scheme 2

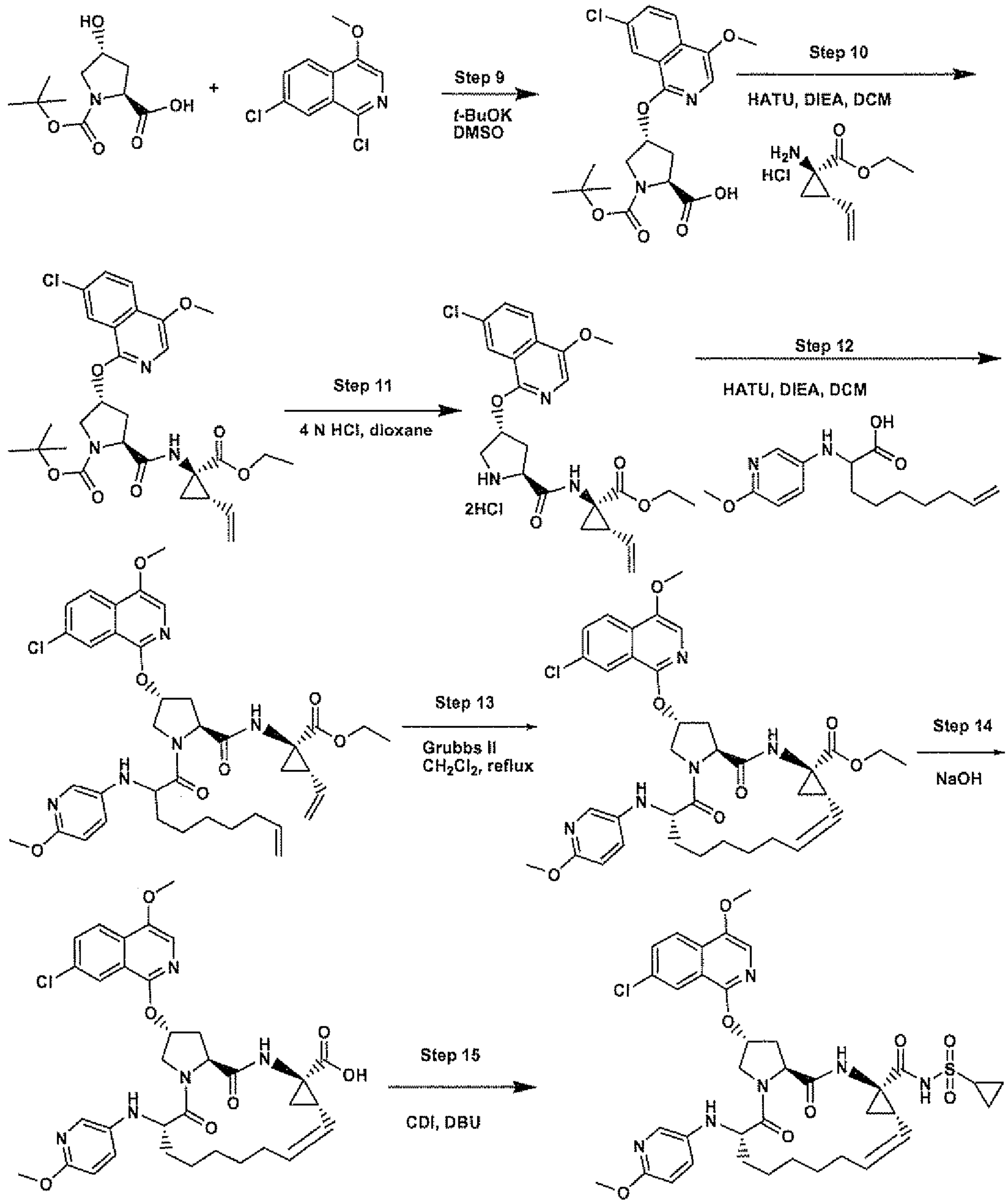


*Step 9:*

To a mixture of 1,7-dichloro-4-methoxyisoquinoline (4.52 g, 20 mmol), Boc-L-Hyp-OH (5.08 g, 22 mmol) and t-BuOK (6.72 g, 60 mmol) was added DMSO (200 mL) with stirring at 10°C and then the resulting slurry was sonicated for 30 min in order to quickly obtain a homogeneous solution at rt. The resulting solution was stirred for 3 h. The reaction mixture was then cooled to 0 °C and quenched with 50 ml of water. The resulting mixture was neutralized/acidified to a final pH of 5 by the addition of 1 N HCl. The resulting mixture was extracted with EtOAc (400 mL), and the organic layer was then washed with brine (200 mL), water (200 mLx2), dried over $MgSO_4$ filtered, and then concentrated *in vacuo* to provide a crude solid (8.36 g). This material was used in the next step without further purification. $^1H$ NMR (400 MHz, $CD_3OD$) $\delta$ ppm 2.34 - 2.47 (m, 1 H), 2.62 - 2.77 (m, 1 H), 3.70 - 3.92 (m, 2 H), 4.42 - 4.59 (m, 1 H), 5.65 (brs, 1 H), 7.54 (s, 1 H), 7.68 (dd, J=8.81, 2.01 Hz, 1 H), 8.02 - 8.13 (m, 2 H); $^{13}C$ NMR (125 MHz, DMSO-$d_6$) (the observed peaks are more than carbon numbers due to Boc rotamers) $\delta$ ppm 13.90, 14.04, 20.71, 22.02, 27.84, 27.98, 30.91, 35.00, 35.87, 51.84, 52.08, 56.21, 57.49, 57.80, 59.70, 73.32, 73.87, 79.14, 79.I9, 119.11, 119.77, 122.3$, 123.35, 128.50, 130.95, 132.25, 145.70, 151.94, 153.25, 153.71, 173.51, 173.98; LC-MS, MS *m/z* 423.

*Step 10:*

A mixture of the product of Step 9, Example 2 (4.23 g, 10 mmol), cyclopropanesulfonamide (2.300 g, 12.00 mmol), Hunig'sBase (6.46 g, 50,0 mmol), and HATU (5.70 g, 15.00 mmol) in $CH_2Cl_2$ (20 mL) was stirred for 4h. After concentration, the residue was purified by Biotage (40+S Si column) eluting with 33% acetone in hexanes to give 4.7 g (84%) of the desired product as a solid. LC-MS, MS m/z 560.

*Step 11:*

To the product of Step 10, Example 2 (5.60 g, 10 mmol) was added 4M HCl (25.00 mL, 100 mmol) in 1,4-dioxane. The formed solution was stirred at 25 °C for 3 h. After concentration under vacuo, to the residue was added ether (20mL), then concentrated again, repeated the procedure 3 times. House vacuum drying gave 5.33 g (100%) of the crude product as a solid, which was used in the next step without further purification.

*Step 12:*

) To the product of Step 11, Example 2 (5.60 g, 10 mmol) was added 4M HCl (25.00 mL, 100 mmol) in 1,4-dioxane. The formed solution was stirred at 25 °C for 3 h. After concentration under vacuo, to the residue was added ether (20mL), then concentrated again, repeated the procedure 3 times. House vacuum drying gave 5.33 g (100%) of the crude product as a solid, which was used in the next step without further purification. LC-MS, MS *mlz* 720.

*Step13:*

A solution of the product of Step 12, Example 2 (160 mg, 0.222 mmol) and GrubbsII (30 mg, 0.035 mmol) in $CH_2Cl_2$ (5 ml) was added refluxed for 16 h. After concentration, the residue was purified by Biotage eluting with 25% acetone in hexanes to give 54 mg of a single diasteremer whose structure was labeled either as shown in Scheme or (*R*)-isomer. LC-MS, MS m/z 692.

*Step 14:*

A solution of the product of Step 13, Example 2 (50 mg, 0.072 mmol) and sodium hydroxide (28.9 mg, 0.722 mmol) in MeOH (5 ml) and water (2.00 ml) was refluxed for 2 h. After concentration, the residue was neutralized with 1 N HCl (1 mL), extracted with EtOAc, dried over $MgSO_4$. Filtration and concentration gave 46 mg of the desired product which was used in the next step without further purification.

*Step 15:*

A solution of the product of Step 14, Example 2 (45 mg, 0.068 mmol) and CDI (15.38 mg, 0.095 mmol) in THF (1 ml) was refluxed for 1 h. After cooling to rt, to the solution was added cyclopropanesulfonamide (13.13 mg, 0.108 mmol) followed by DBU (0,020 ml, 0.136 mmol), then the resulting solution was stirred overnight. After concentration, the residue was purified by prep HPLC to give 15 mg (30%) of the desired product as a white solid. LC-MS, MS *m/z* 767.

*Example 3: Preparation of Compound 3*

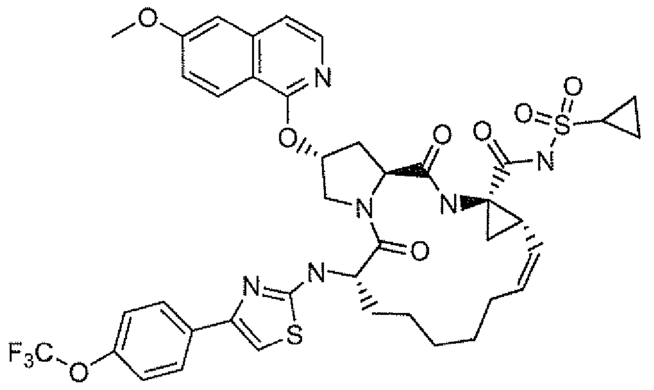

Compound 3

Scheme

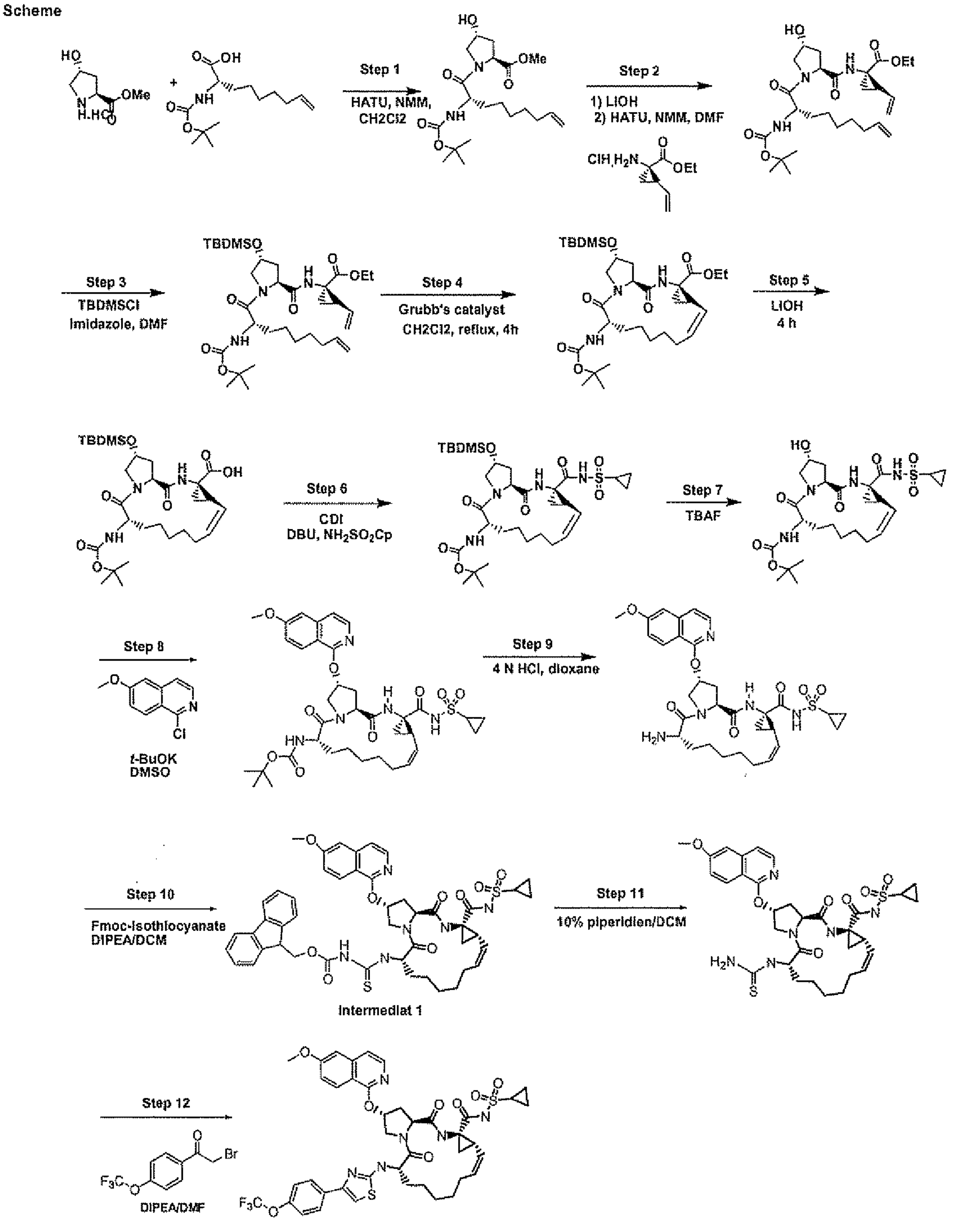


Compound 3

*Step 1: Preparation of 1-(2(S)-tert-Butoxycarbonylamino-non-8-enoyl)-4(R)-hydroxy-pyrrolidine-2(S)-carboxylic acid methyl ester*

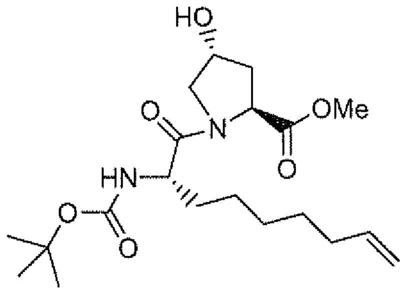

A solution of 2(S)-*tert*-butoxycarbonylamino-8-nonenoic acid (purchased from RSP Amino Acids)(3.5 g, 12.9 mmoL) in 200 mL of DCM was treated sequentially with 4(*R*)-hydroxypyrrolidine-2(*S*)-carboxylic acid methyl ester hydrochloride (2.15 g, 11.8 mmoL), *N*-methyl morpholine (4.25 mL, 38.6 mmol), and HATU (5.37 g, 14.1 mmoL). The reaction mixture was stirred at rt under $N_2$ for 3 days, and then concentrated in vacuo. The residue was partitioned between ethyl acetate and pH 4 buffer (biphthalate). The organic phase was washed with sat. aq. $NaHCO_3$, dried ($MgSO_4$), and concentrated in vacuo to give the crude product. Flash chromatography (50% ethyl acetate/hexane to 100% ethyl acetate) gave 4.7 g (~100%) of 1-(2(S)-tert-Butoxycarbonylamino-non-8-enoyl)-4(*R*)-hydroxy-pyrrolidine-2(S)-carboxylic acid methyl ester as a colorless oil: $^1$H NMR (500 MHz, $CD_3OD$) δ 1.33-1.50(m, 8 H), 1.46 (s, 9 H), 1.57 (m, 1 H), 1.72 (m, 1 H) 2.08 (m, 2 H), 2.28 (m, 1 H), 3.72 (s, 3 H,) 3.75-3.87 (m, 2 H), 4.36 (m, 1 H), 4.51 (bs, 1 H), 4.57 (t, J=8.2 Hz, 1 H), 4.95 (d, *J*=10.4 Hz, 1 H), 5.01 (m, 1 H), 5.83 (m, 1 H). MS *m/z* 399 ($M^+$+1).

*Step 2: Preparation of -1-{[1-(2(S)-tert-Butoxycarbonylamino-non-8-enoyl)-4(R) hydroxy-pyrrolidine-2(S)carbonyl]-(1R)-amino}-2(S)-vinyl-cyclopropanecarboxylic acid ethyl ester*

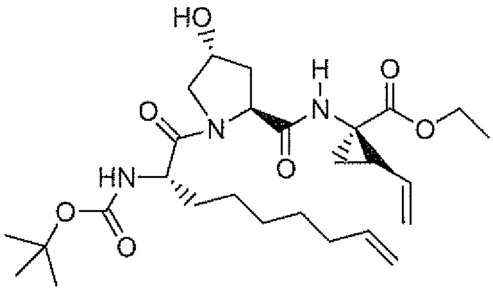

1-(2(*S*)-tert-Butoxycarbonylamino-non-8-enoyl)-4(*R*)-hydroxy-pyrrolidine-2(*S*)-carboxylic acid methyl ester (4.7 g, 11.8 mmoL) was dissolved in THF (80 mL), methanol (20 mL), and water (40 mL). Powdered lithium hydroxide(5.6 g, 233 mmoL) was added. The light yellow slurry was stirred at rt under $N_2$ for 16 h, and then concentrated in vacuo. The residue was partioned between ether and water. The ether phase was discarded, and the aqueous phase was treated with 1N HCl until the pH was 4. This acidic solution was extracted with EtOAc (3x). The combined EtOAc extracts were dried ($MgSO_4$), filtered, and concentrated in vacuo to give 4.36 g (96%) of 1-(2(*S*)-*tert*-butoxycarbonylamino-8-nonenoyl)-4(*R*)-hydroxy-pyrrolidine-2(*S*)-carboxylic acid as a white solid. This acid was then dissolved in 150 mL of DMF and (1*R*,2*S*)-1-amino-2-vinylcyclopropane carboxylic acid ethyl ester hydrochloride (2.61 g, 13.6 mmoL), *N*-methyl morpholine (2.5 mL, 22.6 mmoL), and HATU (5.2 g, 13.7 mmoL) was added. The reaction mixture was stirred at rt under $N_2$ for 16 h, and then concentrated in vacuo. The residue was partitioned between ethyl acetate and pH 4 buffer (biphthalate). The organic phase was washed with sat. aq. $NaHCO_3$, dried ($MgSO_4$), filtered, and concentrated in vacuo to give the crude product. Flash chromatography (60%-80% ethyl acetate/hexane) gave 6.0 g (98%) of 1- {[1-(2(*S*)-*tert*-Butoxycarbonylamino-non-8-enoyl)-4(*R*)-hydroxy-pyrrolidine-2(*S*)carbonyl]-(1*R*)-amino}-2(*S*)-vinyl-cyclopropanecarboxylic acid ethyl ester as a white solid. $^1$H NMR (500 MHz, $CD_3OD$) δ 1.25 (t, *J*=7.2 Hz, 3 H), 1.33-1.80 (m, 10 H), 1.46 (s, 9 H), 2.09 (m, 3 H), 2.25 (m, 2 H), 3.76 (m, 2 H), 4.14 (m, 2 H), 4.27 (dd, *J*=8.5, 5.2 Hz, 1 H), 4.50 (m, 2 H), 4.94 (d, *J*=10.1 Hz, 1 H), 5.01 (dd, *J*=17.1, 1.8 Hz, 1 H), 5.11 (dd, *J*=10.4, 1.8 Hz, 1 H), 5.30 (d, *J*=15.6 Hz, 1 H), 5.80 (m, 2 H), 8.57 (s, 1 H). MS *m/z* 522 ($M^+$+1).

*Step 3: Preparation of 1-{[1-(2-tert-Butoxycarbonylamino-non-8-enoyl)-4-(tert-butyl-dimethyl-silanyloxy)pyrrolidine-2-carbonyl]-amino}-2-vinylcyclopropanecarboxylic acid ethyl ester*

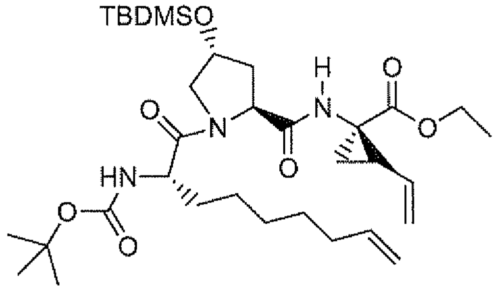

To a mixture of 1-{[1-(2(*S*)-*tert*-Butoxycarbonylamino-non-8-enoyl)-4(*R*)-hydroxy-pyrrolidine-2(*S*)carbonyl]-(1*R*)-amino}-2(*S*)-vinyl-cyclopropanecarboxylic acid ethyl ester (1.5 g, 2.87 mmoL)in 10 mL of DMF was added imidazole (0.25 g, 3.67 mmoL) and tert-butyl-dimethylsilyl chloride (516 mg, 3.44 mmoL). The mixture was stirred at rt for two days. It was then concentrated in vacuo and the residue was dissolved in ethyl acetate. It was then washed with water, dried over Magnesium sulfate, filtered, concentrated *in vacuo* to obtain a solid, which was then purified by flash chromatography (eluting with 20% ethyl acetate in hexane) to isolate a white solid (1.43 g, 78%). $^{1}H$ NMR (300 MHz, $CD_3OD$) $\delta$ 0.10 (s,6 H), 0.89 (s, 9 H), 1.22 (m, 3 H), 1.31-1.48 (m, 16 H), 1.50-1.75 (m, 3 H), 2.06 (m, 3 H), 2.11-2.33 (m, 2 H), 3.70 (m, 2 H), 4.03-4.19 (m, 2 H), 4.21 (m, 1 H), 4.45 (t, *J*=7.87 Hz, 1 H), 4.59 (m, 1 H), 4.91 (d, *J*=9.1 Hz, 1 H), 4.98 (d, *J*=17.20 Hz, 1 H), 5.08 (dd, *J*=10.25, 1.83 Hz, 1 H), 5.27 (dd, *J*=17.38, 1.65 Hz, 1 H), 5.65-5.87 (m, 2 H). MS *m*/*z* 636 ($M^{+}$+1).

*Step 4: Preparation of 14-tert-Butoxycarbonylamino-18-(tert-butyl-dimethyl-silanyloxy)-2,15-dioxo-3,16-diaza-tricyclo[14.3.0.04,6]nonadec-7-ene-4-carboxylic acid ethyl ester*

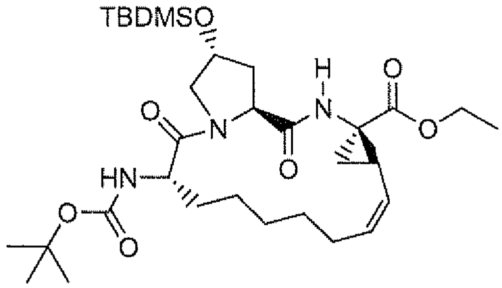

To a solution of 1-{[1-(2-tert-butoxycarbonylamino-non-8-enoyl)-4-(tert-butyl-dimethyl-silanyloxy)-pyrrolidine-2-carbonyl]-amino}-2-vinylcyclopropanecarboxylic acid ethyl ester (1.63 g, 2.56 mmoL) in 640 mL of methylene chloride was added 215 mg (0.26 mmoL) of tricyclohexylphosphine[1,3-bis(2,4,6-tri[benzylidene]ruthenium(IV)dichloride. The mixture was heated at reflux for 15 min. The residue was concentrated in vacuo, and then purified by flash chromatography eluting with 30 % ethyl acetate/hexane. To further decolorize the sample, the crude product was chromatographed a second time eluting with 50% ether in hexane to give 1.5 g (96%) of the product as a white solid. $^{1}H$ NMR (500 MHz, $CD_3Cl$) $\delta$ 0.06 (s, 3 H), 0.07 (s, 3 H), 0.86 (s, 9 H), 1.18-1.24 (m, 6 H), 1.34-1.64 (m, 14 H), 1.86-1.96 (m, 3 H), 2.02-2.09 (m, 1 H), 2.11-2.17 (m, 1 H), 2.19-2.28 (m, 1 H), 2.57-2.63 (m, 1 H), 3.50-3.54 (m, 1 H), 3.71 (dd, *J*=10.22, 6.26 Hz, 1 H), 4.06-4.17 (m, 2 H), 4.52-4.58 (m, 2 H), 4.75 (d, *J*=8.55 Hz, 1 H), 5.21 (t, *J*=9.92 Hz, 1 H), 5.35 (d, *J*=7.63 Hz, 1 H), 5.45-5.50 (m, 1 H), 6.94 (s, 1 H). MS *m*/*z* 608 ($M^{+}$+1).

*Step 5 : Preparation of 14-tert-Butoxycarbonylamino-18-(tert-butyl-dimethyl-silanyloxy)-2,15-dioxo-3,16-diaza-tricyclo[14.3.0.04,6]nonadec-7-ene-4-carboxylic acid*

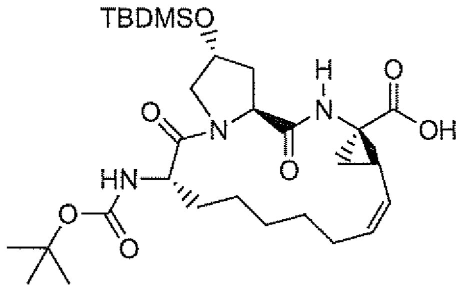

To a solution of 14-tert-Butoxycarbonylamino-18-(tert-butyl-dimethyl-silanyloxy)-2,15-dioxo-3,16-diaza-tricyclo[14.3.0.04,6]nonadec-7-ene-4-carboxylic acid ethyl ester (1.5g, 2.47mmoL) in THF (4 mL), methanol (1 mL), and water (2 mL), was added powdered lithium hydroxide(1.0 g, 50 mmoL), and the light yellow slurry was stirred at rt under $N_2$ for 4 h. The mixture was then concentrated in vacuo and the residue partioned between ether and water. The ether phase was discarded, and the aqueous phase was treated with 1 N HCl until pH 4. This acidic solution was extracted with EtOAc three times. The combined EtOAc extracts were dried ($MgSO_4$) and concentrated in vacuo to give 1.2 g (84%) of an off-white solid. $^1H$ NMR (300 MHz, $CD_3OD$) $\delta$ ppm 0.12 (s, 6 H), 0.89 (s, 9 H), 1.23-1.64 (m, 17 H), 1.70-1.87 (m, 1 H), 1.90-2.49 (m, 6 H), 3.70-3.80 (m,1 H), 3.83-3.90 (m, 1 H), 4.28-4.36 (m, 1 H), 4.47-4.55 (m, 1 H), 4.65 (s, 1 H), 5.30-5.39 (m, 1 H), 5.53-5.62 (m, 1 H). MS *m/z* 580 ($M^+$+1).

*Step 6: preparation of [18-(tert-Butyl-dimethyl-silanyloxy)-4-cyclopropanesulfonylaminocarbonyl-2,15-dioxo-3,16-diaza-tricyclo[14.3.0.04,6]nonadec-7-en-14-yl]-carbamic acid tert-butyl ester*

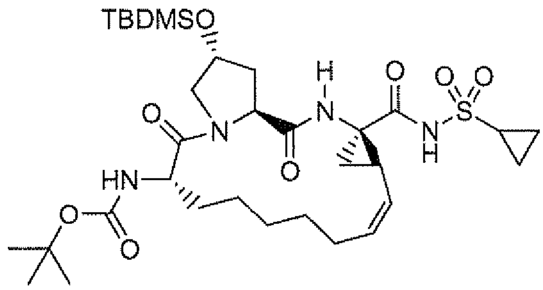

14-tert-Butoxycarbonylamino-18-(tert-butyl-dimethyl-silanyloxy)-2,15-dioxo-3,16-diaza-tricyclo[14.3.0.04,6]nonadec-7-ene-4-carboxylic acid (500 mg, 0.86 mmoL) was dissolved in 25 mL of THF and treated with CDI (180 mg, 1.12 mmoL). (Care was taken to avoid moisture by using oven dried glassware and maintaining a dry N2 atmosphere). After refluxing the reaction mixture for two hours, it was cooled to rt and treated sequentially with cyclopropylsulfonamide (135 mg, 1.12 mmoL) and DBU (170 mg, 1.12 mmoL). After stirring for 4 h at rt, the THF was removed by rotary evaporation. The residue was partitioned between ethyl acetate and pH 4 buffer. The organic phase was dried ($MgSO_4$), filtered, and concentrated in vacuo to give the crude product. It was then purified by flash column, eluting with 33% ethyl acetate in hexane to isolate a white solid (300 mg, 51%). $^1H$ NMR (300 MHz, $CD_3OD$) $\delta$ ppm $^1H$ 0.07 (s, 3 H), 0.08 (s, 3 H), 0.85 (s, 9 H), 0.87-1.49 (m, 21 H), 1.73-1.95 (m, 3 H), 2.08-2.16 (m, 1 H), 2.25-2.36 (m, 2 H), 2.42-2.56 (m, 1 H), 2.85-2.93 (m, 1 H), 3.65-3.74(dd, *J*=10.61, 3.66 Hz, 1 H), 3.89 (d, *J*=10.25 Hz, 1 H), 4.34 (m, *J*=9.70, 9.70 Hz, 1 H), 4.43 (t, *J*=7.87 Hz, 1 H), 4.57 (s, 1 H), 4.94-5.01 (m, 1 H), 5.10 (d, *J*=8.78 Hz, 1 H), 5.66-5.75 (m, 1 H), 6.55 (s, 1 H), 10.13 (s, 1 H). MS *m/z* 683 ($M^+$+1).

*Step 7: (4-Cyclopropanesulfonylaminocarbonyl-18-hydroxy-2,15-dioxo-3,16-diazatricyclo[14.3.0.04,6]nonadec-7-en-14-yl)-carbamic acid tert-butyl ester*

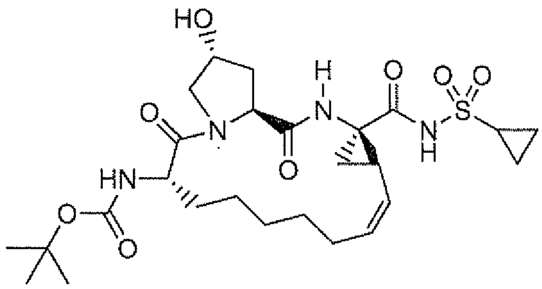

To a mixture of [18-(tert-Butyl-dimethyl-silanyloxy)-4-cyclopropanesulfonylaminocarbonyl-2,15-dioxo-3,16-diazatricyclo[14.3.0.04,5]nonadec-7-en-14-yl]-carbamic acid tert-butyl ester (330mg, 0.48 mmoL) in 25 mL ofTHF was added tetrabutylammonium floride (150 mg, 0.54 mmoL) and the mixture was stirred at rt overnight. THF was removed by rotary evaporation. The residue was partitioned between ethyl acetate and water. The organic phase was dried ($MgSO_4$) and concentrated in vacuo to give the crude product. It was then purified by triturating with hexane to yield a white solid (200 mg, 73%). $^1H$ NMR (500 MHz, $CD_3Cl$) δ ppm 1.87-1.64 (m, 21 H), 1.70-1.98 (m, 3 H), 2.15-2.56 (m, 5 H), 2.85-2.94 (m, 1 H), 3.71 (d, *J*=13.91 Hz, 1 H), 4.10-4.26 (m, 2 H), 4.51 (t, *J*=7.87 Hz, 1 H), 4.62 (s, 1 H), 4.98 (m, 1 H), 5.06 (d, *J*=8.78 Hz, 1 H), 5.64-5.71 (m, 1 H), 6.72 (s, 1 H), 10.24 (s, 1 H). MS *m/z* 569 ($M^+$+1).

*Step 8, Preparation of [4-Cyclopropanesulfonylaminocarbonyl-18-(6-methoxyisoquinolin-1-yloxy)-2,15-dioxo-3,16-diaza-tricyclo[14.3.0.0$^{4,6}$]nonadec-7-en-14-yl]-carbamic acid tert-butyl ester*

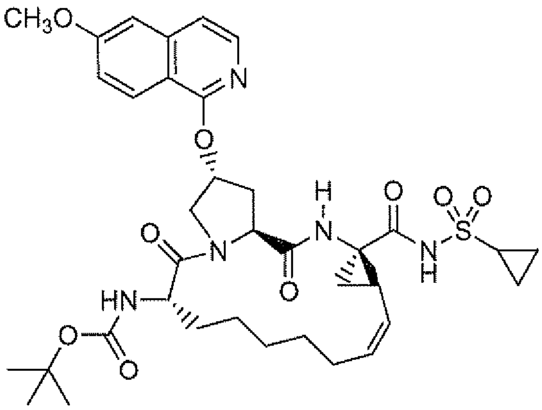

To a mixture of 4-tert-butoxycarbonylamino-18-hydroxy-2,15-dioxo-3,16-diaza-tricyclo[14.3.0.0$^{4,6}$]nonadec-7-ene-4-carboxylic acid (215 mg, 0.46 mmol) in DMSO (5 mL) was added *t*-BuOK (125 mg, 1.11 mmol) and 1-chloro-6-methoxyisoquinoline (110 mg, 0.56 mmol, from Example 1 Scheme 1). The reaction was stirred for 5 h at rt. The reaction mixture then was partitioned between ether (10 mL) and water (10 mL). The aqueous phase was acidified to pH 4 using 1 N HCl. The resulting solution was extracted with EtOAc (3 x 20 mL). The combined EtOAc extracts were dried ($MgSO_4$), filtered, and concentrated in vacuo to give a white solid. Flash chromatography (2% MeOH/$CH_2Cl_2$) gave 140 mg (49%) of the carboxylic acid derivative as a white solid. LC-MS (Method B, retention time: 1.80 min), MS *m/z* 543 ($M^+$+1). The above solid (140 mg, 0.22 mmol) was treated with cyclopropylsulfonamide (35 mg, 0.28 mmol) as described in the general procedure above to give the crude product. Flash chromatography (2% MeOH/DCM) gave 90 mg of the desired product. Further purification by preparative HPLC (YMC Xterra, S5, 30 x 50mm, 50% to 100%B, gradient 9 min, hold 1 min, flow rate 40 mL/min) gave 30 mg (19%) of the product as a white powder. MS *m/z* 726 ($M^+$+1).

*Step 9: Preparation of Cyclopropanesulfonic acid [(Z)-(1S,4R,14S,18R)-14-amino-18-(6-methoxy-isoquinolin-1-yloxy)-2,15-dioxo-3,16-diazatricyclo[14.3.0.0$^{4,6}$]nonadec-7-ene-4-carbonyl]-amide*

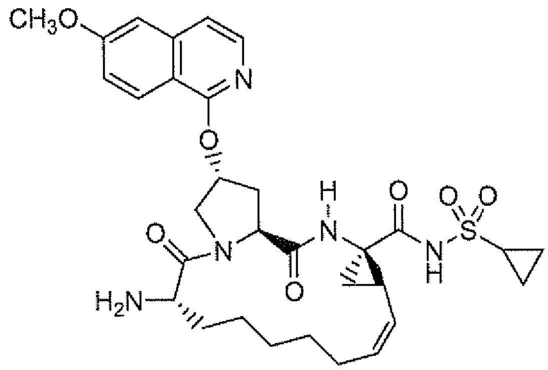

The product of Example 2 (0.944 g, 3.03 mmol) was dissolved in 4M HCl in dioxane (15 mL). The mixture was stirred at room temperature for 4 hours and was then concentrated *in vacuo*. The residue was then dissolved in dichloromethane (20 mL) and again concentrated *in vacuo*. The white solid was subsequently used without further purification. MS *m*/*z* 626 ($M^{+}$+1).

*Step 10: Preparation of intermediate 1*

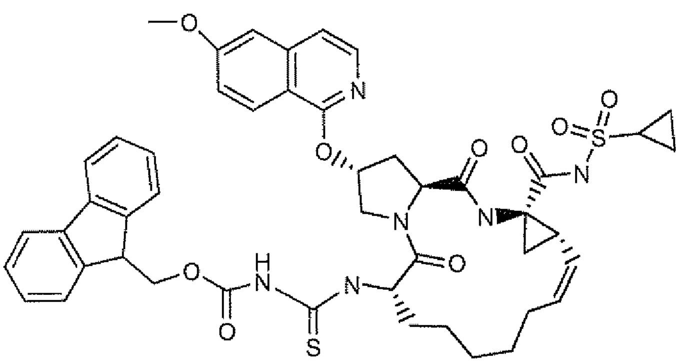

Intermediate 1

Cyclopropanesulfonic acid [(Z)-(1S,4R,14S,18R)-14-amino-18-(6-methoxyisoquinolin-1-yloxy)-2,15-dioxo-3,16-diaza-tricyclo[14.3.0.0$^{4,6}$]nonadec-7-ene-4-carbonyl]-amide(100 mg, 0.16 mmol) dissolved in 5 mL of DCM was treated sequentially with Fmoc-isothiocyanate (49.5 mg, 0.176 mmol, 1.1 eq) and DIPEA (0.084 mL, 0.479 mmol, 3 eq) at rt under $N_2$. The mixture was stirred at rt for 1h. The resulted solution was used directly in the next step.

*Step 11: Preparation of Cyclopropanesulfonic acid [(Z)-(1S,4R,6S,14S,18R)-18-(6-methoxy-isoquinolin-1-yloxy)-2,15-dioxo-14-thioureido-3,16-diazatricyclo[14.3.0.$^{4,6}$]nonadec-7-ene-4-carbonyl]-amide*

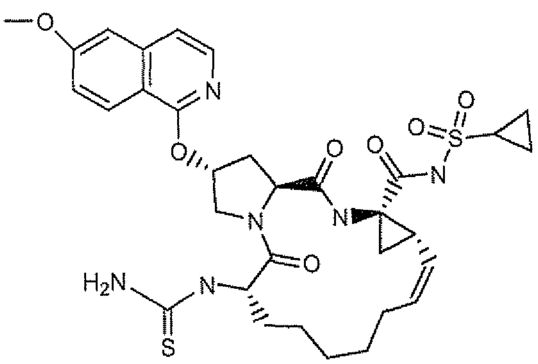

To the reaction solution of intermediate 1(145 mg, 0.16 mmol) in 5 mL DCM was added piperidine(0.5 mL). The reaction mixture was stirred at rt for 2 hs. The solvent was concentrated. The residue was dissolved in EtOAc and washed with HCl 1N and brine solution. Organic layer was dried and concentrated. Flash chromatography (2% MeOH/DCM) gave 57 mg (0.083 mmol, 52%) of the desired product as white solid. MS *m/z* 685.4 ($M^{+}$+1).

*Step 12: Preparation of Compound* 3

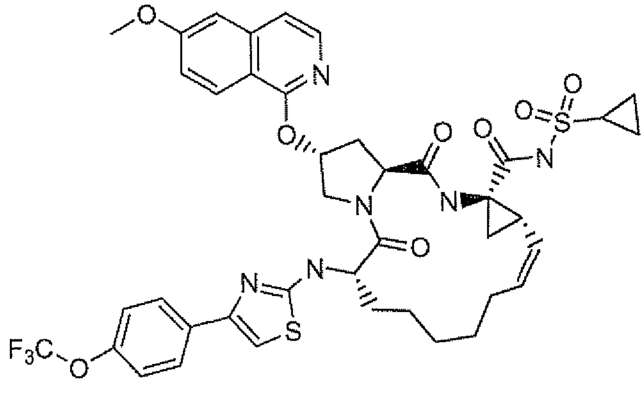

Compound 3

To a mixture of Cyclopropanesulfonic acid [(Z)-(1S,4R,6S,14S,18R)-18-(6-methoxy-isoquinolin-1-yloxy)-2,15-dioxo-14-thioureido-3,16-diazatricyclo[14.3.0.0$^{4,6}$]nonadec-7-ene-4-carbonyl]-amide (20 mg, 0.029 mmol) and DIPEA (0.015 mL, 0.088 mmol, 3eq) in DMF (1ml) was added 2-bromo-1-(4-(trifluoromethoxy)phenyl)ethanone (16.53 mg, 0.058 mmol, 2 eq) at rt under $N_2$. The mixture was stirred at rt for overnight. The reaction mixture was purified by preparative HPLC to give 10.9 mg (0.012 mmol, 43%) of the product as a white powder. $^1$H NMR (400 MHz, $CD_3OD$) δ ppm 0.97 - 1.02 (m, 2 H) 1.04 - 1.11 (m, 4 H) 1.25 - 1.37 (m, 2 H) 1.40 (m, 2 H) 1.57 (m, 2 H) 1.71 (dd, *J*=806, 5.54 Hz, 2 H) 1.92 - 2.03 (m, 2 H) 2.40 (d, *J*=9.06 Hz, 1 H) 2.52 - 2.64 (m, 2 H) 2.83 - 2.93 (m, 2 H) 3.11 (dt, *J*=3.21, 1.54 Hz, 2 H) 3.89 (s, 3 H) 4.21 (dd, *J*=11.71, 3.90 Hz, 1 H) 4.63 (t, *J*=8.06 Hz, 1 H) 4.75 - 4.81 (m, 2 H) 5.08 (d, *J*=9.57 Hz, 1 H) 5.70 (d, *J*=10.07 Hz, 1 H) 6.02 (s, 1 H) 6.73 (s, 1 H, NH) 6.83 - 6.89 (m, 3 H) 7.12 (d, *J*=2.27 Hz, 1 H) 7.21 (d, *J*=6.04 Hz, 1 H) 7.60 - 7.67 (m, 2 H) 7.79 (d, *J*=6.04 Hz, 1 H) 9.00 (s, 1 H, NH). MS *m/z* 869.3 ($M^{+}$+1).

*Example 4: Preparation of Compound 4*

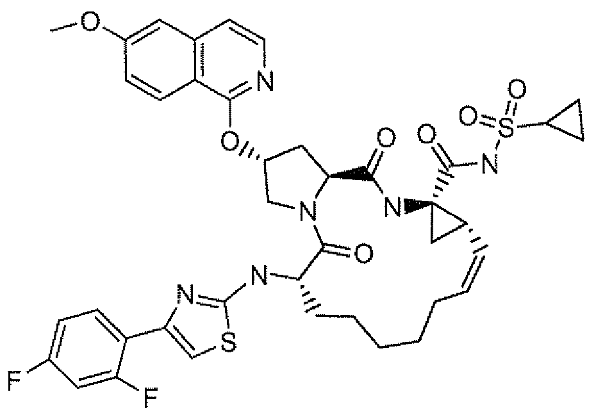

Compound 4

Example 4 was prepared by the same procedure as described for the preparation of Example 3, except 2-bromo-1-(2,4-difluorophenyl)ethanone was used instead of 2-bromo-1-(4-(trifluoromethoxy)phenyl)ethanone at Step 12.

$^1H$ NMR (400 MHz, $CD_3OD$) δ ppm 0.96 - 1.02 (m, 1 H) 1.04 - 1.11 (m, 2 H) 1.25 - 1.31 (m, 1 H) 1.36 (m, 1 H) 1.58 (dd, *J*=9.44, 5.41 Hz, 4 H) 1.71 (dd, *J*=8.06, 5.54 Hz, 2 H) 1.91 - 2.03 (m, 2 H) 2.38 - 2.47 (m, 1 H) 2.50 - 2.61 (m, 1 H) 2.64 (m, 1 H) 2.85 - 2.95 (m, 1 H) 3.64 (s, 2 H) 3.90 (s, 3 H) 4.17 (dd, *J*=11.33, 3.78 Hz, 1 H) 4.63 (t, *J*=8.18 Hz, 1 H) 4.74 (dd, *J*=10.58, 3.27 Hz, 1 H) 4.88 - 4.96 (m, 2 H) 5.04 - 5.10 (m, 1 H) 5.66 - 5.73 (m, 1 H) 6.05 (s, 1 H) 6.45 (td, *J*=8.31, 2.27 Hz, 1 H) 6.63 (s, 1 H, NH) 6.65 - 6.69 (m, 1 H), 6.89 (dd, *J*=9.06, 2.52 Hz, 1 H) 7.12 (d, *J*=2.52 Hz, 1 H) 7.19 (d, *J*=5.79 Hz, 1 H) 7.58 (d, *J*=9.06 Hz, 1 H) 7.74 - 7.81 (m, 2 H) 8.98 (s, 1 H, NH) MS *m/z* 821.6 ($M^+$+H).

*Example 5: Preparation of Compound 5*

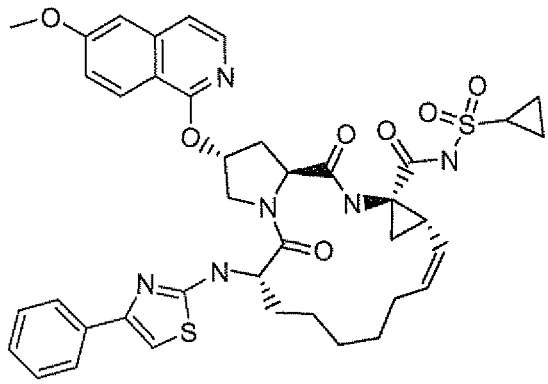

Compound 5

Example 5 was prepared by the same procedure as described for the preparation of Example 3, except 2-bromo-1-phenylethanone was used instead of 2-bromo-1-(4-(trifluoromethoxy)phenyl)ethanone at Step 12. $^1H$ NMR (400 MHz, $CD_3OD$) δ ppm 0.98 - 1.03 (m, 1 H) 1.05 - 1.12 (m, 2 H) 1.26 - 1.38 (m, 4 H) 1.47 - 1.59 (m, 2 H) 1.72 (dd, *J*=8.18, 5.67 Hz, 1 H) 1.81 (s, 1 H) 2.02 (s, 2 H) 2.36 (t, *J*=8.94 Hz, 1 H) 2.55 (ddd, *J*=13.85, 8.69, 4.66 Hz, 2 H) 2.63 - 2.71 (m, 1 H) 2.91 (tt, *J*=7.93, 4.91 Hz, 1 H) 3.64 (s, 3 H) 3.89 (s, 3 H) 4.18 (dd, *J*=11.58, 3.78 Hz, 1 H) 4.57 (s, 1 H) 4.69 - 4.76 (m, 2 H) 5.01 - 5.11 (m, 2 H) 5.66 - 5.74 (m, 1 H) 5.98 (s, 1 H) 6.63 (s, 1 H, NH) 7.01 (dd, *J*=9.19, 2.39 Hz, 1 H) 7.16 (d, *J*=2.52 Hz, 1 H) 7.19 - 7.26 (m, 4 H) 7.53 (dd, *J*=7.93, 1.64 Hz, 2 H) 7.84 (d, *J*=6.04 Hz, 2 H) 9.09 (s, 1 H, NH) MS *m/z* 785.6 ($M^+$+ H).

*Example 6: Preparation of Compound 6*

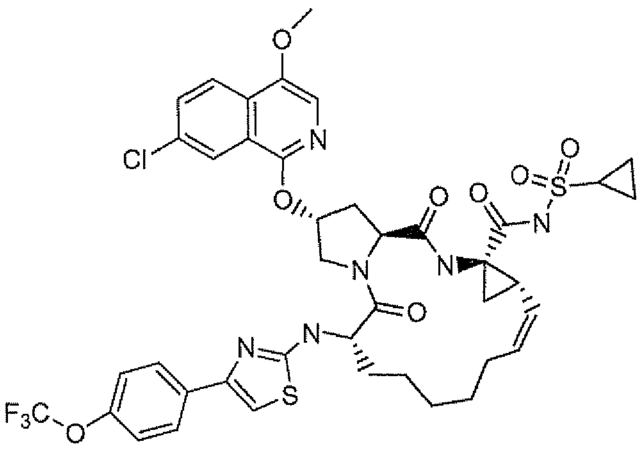

Compound 6

Example 6 was prepared by the same procedure as described for the preparation of Example 3, except 1,7-dichloro-4-methoxy-isoquinoline (from Example 2 Scheme 1) was used instead of isoquinoline chloride at Step 8. $^1$H NMR (400 MHz, $MCD_3OD$) δ ppm 0.99 (ddd, *J*=11.90, 8.25, 3.27 Hz, 1 H) 1.03 - 1.13 (m, 2 H) 1.24 1.36 (m, 2 H) 1.58 (dd, *J*=9.44, 5.16 Hz, 4 H) 1.70 (dd, *J*=8.18, 5.41 Hz, 2 H) 1.89 - 1.98 (m, 1 H) 2.02 (s, 1 H) 2.41 - 2.52 (m, 3 H) 2.64 (s, 1 H) 2.84 - 2.92 (m, 1 H) 3.31 - 3.34 (m, 2 H) 3.64 (s, 1 H) 3.92 (s, 3 H) 4.15 (dd, *J*=11.33, 3.78 Hz, 1 H) 4.58 (t, *J*=8.31 Hz, 1 H) 4.78 (dd, *J*=10.95, 3.15 Hz, 2 H) 5.03 - 5.13 (m, 2 H) 5.68 (m, 1 H) 6.08 (s, 1 H) 6.68 (m, 2H) 6.75 (s,1 H) 7.40 (s, 1 H, NH) 7.46 (s, 1 H) 7.52 (dd, *J*=8.81, 2.27 Hz, 1 H) 7.56 - 7.62 (m, 2 H) 7.93 (d, *J*=8.81 Hz, 1 H) 8.93 (s, 1 H, NH). MS *m*/*z* 903.3 ($M^+$+ H).

*Biological Studies*

HCV NS3/4A protease complex enzyme assays and cell-based HCV replicon assays were utilized in the present disclosure, and were prepared, conducted and validated as follows:

*Generation of recombinant HCV NS3/4A protease complex*

HCV NS3 protease complexes, derived from the BMS strain, H77 strain or J4L6S strain, were generated, as described below. These purified recombinant proteins were generated for use in a homogeneous assay (see below) to provide an indication of how effective compounds of the present disclosure would be in inhibiting HCV NS3 proteolytic activity.

Serum from an HCV-infected patient was obtained from Dr. T. Wright, San Francisco Hospital. An engineered full-length cDNA (compliment deoxyribonucleic acid) template of the HCV genome (BMS strain) was constructed from DNA fragments obtained by reverse transcription-PCR (RT-PCR) of serum RNA (ribonucleic acid) and using primers selected on the basis of homology between other genotype 1 a strains. From the determination of the entire genome sequence, a genotype 1a was assigned to the HCV isolate according to the classification of Simmonds et al. (See P Simmonds, KA Rose, S Graham, SW Chan, F McOmish, BC Dow, EA Follett, PL Yap and H Marsden, J. Clin. Microbiol., 31(6), 1493-1503 (1993)). The amino acid sequence of the nonstructural region, NS2-5B, was shown to be >97% identical to HCV genotype 1a (H77) and 87% identical to genotype 1b (J4L6S). The infectious clones, H77 (1a genotype) and J4L6S (1b genotype) were obtained from R. Purcell (NIH) and the sequences are published in Genbank (AAB67036, see Yanagi,M., Purcell,R.H., Emerson,S.U. and Bukh,J. Proc. Natl. Acad. Sci. U.S.A. 94(16),8738-8743 (1997); AF054247, see Yanagi,M., St Claire,M., Shapiro,M., Emerson,S.U., Purcell,R.H. and Bukh,J, Virology 244 (1), 161-172. (1998)).

The H77 and J4L6S strains were used for production of recombinant NS3/4A protease complexes. DNA encoding the recombinant HCV NS3/4A protease complex (amino acids 1027 to 1711) for these strains were manipulated as described by P. Gallinari et al. (see Gallinari P, Paolini C, Brennan D, Nardi C, Steinkuhler C, De Francesco R. Biochemistry. 38(17):5620-32, 1999)). Briefly, a three-lysine solubilizing tail was added at the 3'-end of the NS4A coding region. The cysteine in the P1 position of the NS4A-NS4B cleavage site (amino acid 1711) was changed to a glycine to avoid the proteolytic cleavage of the lysine tag. Furthermore, a cysteine to serine mutation was introduced by PCR at amino acid position 1454 to prevent the autolytic cleavage in the NS3 helicase domain. The variant DNA fragment was cloned in the pET21b bacterial expression vector (Novagen) and the NS3/4A complex was expressed in Escherichia. coli strain BL21 (DE3) (Invitrogen) following the protocol described by P. Gallinari et al. (see Gallinari P, Brennan D, Nardi C, Brunetti M, Tomei L, Steinkuhler C, De Francesco R., J Virol. 72(8):6758-69 (1998)) with modifications. Briefly, the NS3/4A protease complex expression was induced with 0.5 millimolar (mM) Isopropyl β-D-1-thiogalactopyranoside (IPTG) for 22 hours (h) at 20°C. A typical fermentation (1 Liter (L)) yielded approximately 10 grams (g) of wet cell paste. The cells were resuspended in lysis buffer (10 mL/g) consisting of 25 mM *N*-(2-Hydroxyethyl)Piperazine-*N*'-(2-Ethane Sulfonic acid) (HEPES), pH 7.5, 20% glycerol, 500 mM Sodium Chloride (NaCl), 0.5% Triton X-100, 1 microgram/milliliter ("μg/mL") lysozyme, 5 mM Magnesium Chloride ($MgCl_2$), 1 μg/ml Dnasel, 5 mM β-Mercaptoethanol (βME), Protease inhibitor-Ethylenediamine Tetraacetic acid (EDTA) free (Roche), homogenized and incubated for 20 minutes (min) at 4°C. The homogenate was sonicated and clarified by ultra-centrifugation at 235000 g for 1 hour (h) at 4°C. Imidazole was added to the supernatant to a final concentration of 15 mM and the pH adjusted to 8.0. The crude protein extract was loaded on a Nickel-Nitrilotriacetic acid (Ni-NTA) column pre-equilibrated with buffer B (25 mM HEPES, pH 8.0, 20% glycerol, 500 mM NaCl, 0.5% Triton X-100, 15 mM imidazole, 5 mM βME). The sample was loaded at a flow rate of 1 mL/min. The column was washed with 15 column volumes of buffer C (same as buffer B except with 0.2% Triton X-100). The protein was eluted with 5 column volumes of buffer D (same as buffer C except with 200 mM Imidazole).

NS3/4A protease complex-containing fractions were pooled and loaded on a desalting column Superdex-S200 pre-equilibrated with buffer D (25 mM HEPES, pH 7.5, 20% glycerol, 300 mM NaCl, 0.2% Triton X-100, 10 mM βME). Sample was loaded at a flow rate of 1 mL/min. NS3/4A protease complex-containing fractions were pooled and concentrated to approximately 0.5 mg/ml. The purity of the NS3/4A protease complexes, derived from the BMS, H77 and J4L6S strains, were judged to be greater than 90% by SDS-PAGE and mass spectrometry analyses. The enzyme was

stored at -80 °C, thawed on ice and diluted prior to use in assay buffer.

*FRET peptide assay to monitor HCV NS3/4A proteolytic activty*

The purpose of this in vitro assay was to measure the inhibition of HCV NS3 protease complexes, derived from the BMS strain, H77 strain or J4L6S strain, as described above, by compounds of the present disclosure. This assay provides an indication of how effective compounds of the present disclosure would be in inhibiting HCV NS3 proteolytic activity.

In order to monitor HCV NS3/4A protease activity, an NS3/4A peptide substrate was used. The substrate was RET S1 (Resonance Energy Transfer Depsipeptide Substrate; AnaSpec, Inc. cat # 22991)(FRET peptide), described by Taliani et al. in Anal. Biochem. 240(2):60-67 (1996). The sequence of this peptide is loosely based on the NS4A/NS4B natural cleavage site for the HCV NS3 protease except there is an ester linkage rather than an amide bond at the cleavage site. The peptide also contains a fluorescence donor, EDANS, near one end of the peptide and an acceptor, DABCYL, near the other end. The fluorescence of the peptide is quenched by intermolecular resonance energy transfer (RET) between the donor and the acceptor, but as the NS3 protease cleaves the peptide the products are released from RET quenching and the fluorescence of the donor becomes apparent.

The peptide substrate was incubated with one of the three recombinant NS3/4A protease complexes, in the absence or presence of a compound of the present disclosure. The inhibitory effects of a compound were determined by monitoring the formation of fluorescent reaction product in real time using a Cytofluor Series 4000.

The reagents were as follow: HEPES and Glycerol (Ultrapure) were obtained from GIBCO-BRL. Dimethyl Sulfoxide (DMSO) was obtained from Sigma. β-Mercaptoethanol was obtained from Bio Rad.

Assay buffer: 50 mM HEPES, pH 7.5; 0.15 M NaCl; 0.1% Triton; 15% Glycerol; 10 mM βME. Substrate: 2 μM final concentration (from a 2 mM stock solution in DMSO stored at -20°C). HCV NS3/4A protease type 1a (1b), 2-3 nM final concentration (from a 5 μM stock solution in 25 mM HEPES, pH 7.5, 20% glycerol, 300 mM NaCl, 0.2% Triton-X100, 10 mM βME). For compounds with potencies approaching the assay limit, the assay was made more sensitive by adding 50 μg/ml Bovine Serum Albumin (Sigma) to the assay buffer and reducing the end protease concentration to 300 pM.

The assay was performed in a 96-well polystyrene black plate from Falcon. Each well contained 25 μl NS3/4A protease complex in assay buffer, 50 μl of a compound of the present disclosure in 10% DMSO/assay buffer and 25 μl substrate in assay buffer. A control (no compound) was also prepared on the same assay plate. The enzyme complex was mixed with compound or control solution for 1 min before initiating the enzymatic reaction by the addition of substrate. The assay plate was read immediately using the Cytofluor Series 4000 (Perspective Biosystems). The instrument was set to read an emission of 340 nm and excitation of 490 nm at 25°C. Reactions were generally followed for approximately 15 min.

The percent inhibition was calculated with the following equation:

$$100-[(\delta F_{inh}/\delta F_{con})x100]$$

where δF is the change in fluorescence over the linear range of the curve. A non-linear curve fit was applied to the inhibition-concentration data, and the 50% effective concentration ($IC_{50}$) was calculated by the use of Excel XLfit software using the equation, y=A+((B-A)/(1+((C/x)^D))).

All of the compounds tested were found to inhibit the activity of the NS3/4A protease complex with IC50's of 7 nM or less. Further, compounds of the present disclosure, which were tested against more than one type of NS3/4A complex, were found to have similar inhibitory properties though the compounds uniformly demonstrated greater potency against the 1b strains as compared to the 1a strains.

*Specificity Assays*

The specificity assays were performed to demonstrate the in vitro selectivity of the compounds of the present disclosure in inhibiting HCV NS3/4A protease complex as compared to other serine or cysteine proteases.

The specificities of compounds of the present disclosure were determined against a variety of serine proteases: human neutrophil elastase (HNE), porcine pancreatic elastase (PPE) and human pancreatic chymotrypsin and one cysteine protease: human liver cathepsin B. In all cases a 96-well plate format protocol using a fluorometric Amino-Methyl-Coumarin (AMC) substrate specific for each enzyme was used as described previously (PCT Patent Application No. WO 00/09543) with some modifications to the serine protease assays. All enzymes were purchased from Sigma, EMDbiosciences while the substrates were from Bachem, Sigma and EMDbiosciences.

Compound concentrations varied from 100 to 0.4 μM depending on their potency. The enzyme assays were each initiated by addition of substrate to enzyme-inhibitor pre-incubated for 10 min at room temperature and hydrolysis to 15% conversion as measured on cytofluor.

The final conditions for each assay were as follows:

50 mM Tris(hydroxymethyl) aminomethane hydrochloride (Tris-HCl) pH 8, 0.5 M Sodium Sulfate ($Na_2SO_4$), 50 mM NaCl, 0.1 mM EDTA, 3% DMSO, 0.01% Tween-20 with 5 μM LLVY-AMC and 1 nM Chymotrypsin.

50mM Tris-HCl, pH 8.0, 50 mM NaCl, 0.1mM EDTA, 3% DMSO, 0.02% Tween-20, 5 μM succ-AAPV-AMC and 20 nM HNE or 8 nM PPE;

100 mM NaOAC (Sodium Acetate) pH 5.5, 3% DMSO, 1 mM TCEP (Tris(2-carboxyethyl)phosphine hydrochloride), 5 nM Cathepsin B (enzyme stock activated in buffer containing 20 mM TCEP before use), and 2 μM Z-FR-AMC diluted in $H_2O$.

The percentage of inhibition was calculated using the formula:

$$[1-((UV_{inh}-UV_{blank})/(UV_{ctl}-UV_{blank}))] \times 100$$

A non-linear curve fit was applied to the inhibition-concentration data, and the 50% effective concentration ($IC_{50}$) was calculated by the use of Excel XLfit software.

*Generation of HCV Replicon*

An HCV replicon whole cell system was established as described by Lohmann V, Korner F, Koch J, Herian U, Theilmann L, Bartenschlager R., Science 285(5424):110-3 (1999). This system enabled us to evaluate the effects of our HCV Protease compounds on HCV RNA replication. Briefly, using the HCV strain 1b sequence described in the Lohmann paper (Assession number:AJ238799), an HCV cDNA was synthesized by Operon Technologies, Inc. (Alameda, CA), and the full-length replicon was then assembled in plasmid pGem9zf(+) (Promega, Madison, WI) using standard molecular biology techniques. The replicon consists of (i) the HCV 5' UTR fused to the first 12 amino acids of the capsid protein, (ii) the neomycin phosphotransferase gene (neo), (iii) the IRES from encephalomyocarditis virus (EMCV), and (iv) HCV NS3 to NS5B genes and the HCV 3' UTR. Plasmid DNAs were linearized with ScaI and RNA transcripts were synthesized in vitro using the T7 MegaScript transcription kit (Ambion, Austin, TX) according to manufacturer's directions. In vitro transcripts of the cDNA were transfected into the human hepatoma cell line, HUH-7. Selection for cells constitutively expressing the HCV replicon was achieved in the presence of the selectable marker, neomycin (G418). Resulting cell lines were characterized for positive and negative strand RNA production and protein production over time.

*HCV Replicon FRET Assay*

The HCV replicon FRET assay was developed to monitor the inhibitory effects of compounds described in the disclosure on HCV viral replication. HUH-7 cells, constitutively expressing the HCV replicon, were grown in Dulbecco's Modified Eagle Media (DMEM) (Gibco-BRL) containing 10% Fetal calf serum (FCS) (Sigma) and 1 mg/ml G418 (Gibco-BRL). Cells were seeded the night before (1.5 x $10^4$ cells/well) in 96-well tissue-culture sterile plates. Compound and no compound controls were prepared in DMEM containing 4% FCS, 1:100 Penicillin/Streptomysin (Gibco-BRL), 1:100 L-glutamine and 5% DMSO in the dilution plate (0.5% DMSO final concentration in the assay). Compound/DMSO mixes were added to the cells and incubated for 4 days at 37°C. After 4 days, cells were first assessed for cytotoxicity using alamar Blue (Trek Diagnotstic Systems) for a $CC_{50}$ reading. The toxicity of compound ($CC_{50}$) was determined by adding 1/10$^{th}$ volume of alamar Blue to the media incubating the cells. After 4 h, the fluorescence signal from each well was read, with an excitation wavelength at 530 nm and an emission wavelength of 580 nm, using the Cytofluor Series 4000 (Perspective Biosystems). Plates were then rinsed thoroughly with Phosphate-Buffered Saline (PBS) (3 times 150 μl). The cells were lysed with 25 μl of a lysis assay reagent containing an HCV protease substrate (5X cell Luciferase cell culture lysis reagent (Promega #E153A) diluted to 1X with distilled water, NaCl added to 150 mM final, the FRET peptide substrate (as described for the enzyme assay above) diluted to 10 μM final from a 2 mM stock in 100% DMSO. The plate was then placed into the Cytofluor 4000 instrument which had been set to 340 nm excitation/490 nm emission, automatic mode for 21 cycles and the plate read in a kinetic mode. $EC_{50}$ determinations were carried out as described for the $IC_{50}$ determinations.

*HCV Replicon Luciferase Reporter Assay*

As a secondary assay, $EC_{50}$ determinations from the replicon FRET assay were confirmed in a replicon luciferase reporter assay. Utilization of a replicon luciferase reporter assay was first described by Krieger et al (Krieger N, Lohmann V, and Bartenschlager R, J. Virol. 75(10):4614-4624 (2001)). The replicon construct described for our FRET assay was modified by inserting cDNA encoding a humanized form of the Renilla luciferase gene and a linker sequence fused directly to the 3'-end of the luciferase gene. This insert was introduced into the replicon construct using an Ascl restriction site located in core, directly upstream of the neomycin marker gene. The adaptive mutation at position 1179 (serine to isoleucine) was also introduced (Blight KJ, Kolykhalov, AA, Rice, CM, Science 290(5498):1972-1974). A stable cell line constitutively expressing this HCV replicon construct was generated as described above. The luciferase reporter assay was set up as described for the HCV replicon FRET assay with the following modifications. Following 4 days in a 37 °C15% $CO_2$ incubator, cells were analyzed for Renilla Luciferase activity using the Promega Dual-Glo Luciferase Assay System. Media (100 μl) was removed from each well containing cells. To the remaining 50 μl of media, 50 μl of Dual-Glo Luciferase Reagent was added, and plates rocked for 10 min to 2 h at room temperature. Dual-Glo Stop & Glo Reagent (50 μl) was then added to each well, and plates were rocked again for an additional 10 min to 2 h at room temperature. Plates were read on a Packard TopCount NXT using a luminescence program.
The percentage inhibition was calculated using the formula below:

$$\% \text{ control} = \frac{\text{average luciferase signal in experimental wells (+ compound)}}{\text{average luciferase signal in DMSO control wells (- compound)}}$$

The values were graphed and analyzed using XLfit to obtain the $EC_{50}$ value.

Representative compounds of the disclosure were assessed in the HCV enzyme assays, HCV replicon cell assay and/or in several of the outlined specificity assays. For example, Compound 1 was found to have an $IC_{50}$ of 8.7 nanomolar (nM) against the NS3/4A BMS strain in the enzyme assay. Similar potency values were obtained with the published H77 ($IC_{50}$ of 1.9 nM) and J4L6S ($IC_{50}$ of 1.2 nM) strains. The $EC_{50}$ value in the replicon FRET assay was 21 nM.

In the specificity assays, the same compound was found to have the following activity: HLE = 1.5 μM; PPE > 25 μM; Chymotrypsin = 25 μM; Cathepsin B >25 μM. These results indicate this family of compounds is highly specific for the NS3 protease and many of these members inhibit HCV replicon replication.

The compounds of the current disclosure were tested and found to have activities as follows:

*Table 2*

| *Compound Number* | *IC50* | *EC50* |
|---|---|---|
| 1 | 7 | 20.5 |
| 2 | 6 | 9.1 |
| 3 | 5 | 6.2 |
| 4 | 11 | 13.8 |
| 5 | 3 | 8.1 |
| 6 | 2 | 8.7 |

## Claims

**1.** A compound of formula (I)

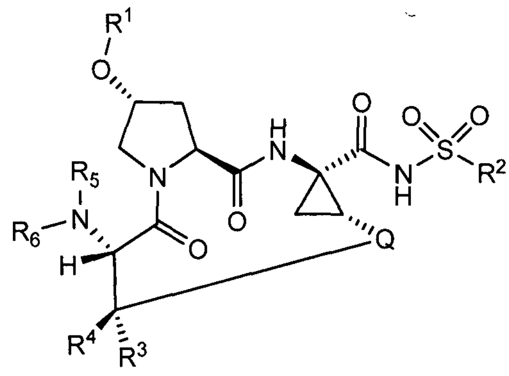

(I),

or a pharmaceutically acceptable salt thereof, wherein

Q is a $C_{3-9}$ saturated or unsaturated chain optionally containing from one to three heteroatoms independently selected from O, $S(O)_m$, and $NR^8$; wherein m is 0, 1, or 2, and $R^8$ is selected from hydrogen, alkoxy, alkoxycarbonyl, alkyl, alkylcarbonyl, alkylsulfonyl, aminocarbonyl, arylsulfonyl, cycloalkyl, (cycloalkyl)alkyl, cycloalkyloxy, dialkylaminocarbonyl, dialkylaminocarbonylalkyl, haloalkyl, and heterocyclylcarbonyl;
$R^1$ is isoquinolinyl optionally substituted with one or two $R^7$ groups;
$R^2$ is selected from alkyl, aryl, cycloalkyl, (cycloalkyl)alkyl, heterocyclyl, and $-NR^aR^b$, wherein the alkyl, the cycloalkyl and the cycloalkyl part of the (cycloalkyl)alkyl are optionally substituted with one, two, or three substituents selected from alkenyl, alkoxy, alkoxyalkyl, alkyl, arylalkyl, arylcarbonyl, cyano, cycloalkenyl, (cycloalkyl)alkyl, halo, haloalkoxy, haloalkyl, and $(NR^eR^f)$carbonyl;
$R^3$ and $R^4$ are independently selected from hydrogen, alkoxyalkyl, alkyl, haloalkoxyalkyl, and haloalkyl;
$R^5$ is selected from hydrogen, alkyl and haloalkyl;
$R^6$ is selected from phenyl and a five- or six-membered partially or fully unsaturated ring optionally containing one, two, three, or four heteroatoms selected from nitrogen, oxygen, and sulfur; wherein each of the rings is optionally substituted with one, two, three, or four substitutents independently selected from alkoxy, alkoxycarbonyl, alkyl, alkylcarbonyl, alkylsulfanyl, carboxy, cyano, cycloalkyl, cycloalkyloxy, halo, haloalkyl, haloalkoxy, $-NR^cR^d$, $(NR^eR^f)$carbonyl, $(NR^eR^f)$sulfonyl, and oxo; provided that when $R^6$ is a six-membered substituted ring all substituents on the ring other than fluoro must be in the meta and/or para positions relative to the ring's point of attachment to the parent molecular moiety;
each $R^7$ is independently selected from alkoxy, alkoxycarbonyl, alkyl, alkylcarbonyl, aryl, carboxy, cyano, cyanoalkyl, cycloalkyl, halo, haloalkyl, haloalkoxy, heterocyclyl, hydroxy, hydroxyalkyl, nitro,$-NR^cR^d$, $(NR^cR^d)$alkyl, $(NR^cR^d)$alkoxy, $(NR^eR^f)$carbonyl, and $(NR^eR^f)$sulfonyl; or
two adjacent $R^7$ groups, together with the carbon atoms to which they are attached, form a four- to seven-membered partially- or fully-unsaturated ring optionally containing one or two heteroatoms independently selected from nitrogen, oxygen, and sulfur, wherein the ring is optionally substituted with one, two, or three groups independently selected from alkoxy, alkyl, cyano, halo, haloalkoxy, and haloalkyl;
$R^a$ and $R^b$ are independently selected from hydrogen, alkoxy, alkyl, aryl, arylalkyl, cycloalkyl, (cycloalkyl)alkyl, heterocyclyl, and heterocyclylalkyl; or $R^a$ and $R^b$ together with the nitrogen atom to which they are attached form a four to seven-membered monocyclic heterocyclic ring;
$R^c$ and $R^d$ are independently selected from hydrogen, alkoxyalkyl, alkoxycarbonyl, alkyl, alkylcarbonyl, arylalkyl, and haloalkyl;
$R^e$ and $R^f$ are independently selected from hydrogen, alkyl, aryl, arylalkyl, and heterocyclyl; wherein the aryl, the aryl part of the arylalkyl, and the heterocyclyl are optionally substituted with one or two substituents independently selected from alkoxy, alkyl, and halo; and
$R^g$ and $R^h$ are independently selected from hydrogen, alkyl, aryl, arylalkyl, cycloalkyl, (cycloalkyl)alkyl, heterocyclyl, and heterocyclyl; or $R^g$ and $R^h$ together with the nitrogen atom to which they are attached form a monocyclic heterocyclic ring wherein the monocyclic heterocyclic ring is optionally fused to a phenyl ring to form a bicyclic system; wherein the monocyclic heterocyclic ring and the bicyclic system are optionally substituted with one, two, or three substituents independently selected from alkoxy, alkyl, halo, haloalkoxy, and haloalkyl.

**2.** A compound of formula (I)

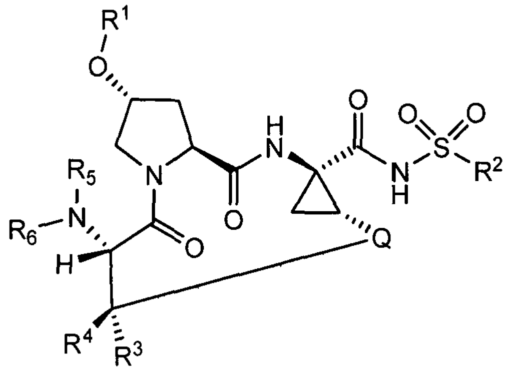

(I),

or a pharmaceutically acceptable salt thereof, wherein

Q is a $C_{3-9}$ saturated or unsaturated chain optionally containing from one to three heteroatoms independently selected from O, $S(O)_m$, and $NR^8$; wherein m is 0, 1, or 2, and $R^8$ is selected from hydrogen, alkoxy, alkoxycarbonyl, alkyl, alkylcarbonyl, alkylsulfonyl, aminocarbonyl, arylsulfonyl, cycloalkyl, (cycloalkyl)alkyl, cycloalkyloxy, dialkylaminocarbonyl, dialkylaminocarbonylalkyl, haloalkyl, and heterocyclylcarbonyl;
$R^1$ is isoquinolinyl optionally substituted with one or two $R^7$ groups;
$R^2$ is selected from alkyl, aryl, cycloalkyl, (cycloalkyl)alkyl, heterocyclyl, and $-NR^aR^b$, wherein the alkyl, the cycloalkyl and the cycloalkyl part of the (cycloalkyl)alkyl are optionally substituted with one, two, or three substituents selected from alkenyl, alkoxy, alkoxyalkyl, alkyl, arylalkyl, arylcarbonyl, cyano, cycloalkenyl, (cycloalkyl)alkyl, halo, haloalkoxy, haloalkyl, and $(NR^eR^f)$carbonyl;
$R^3$ and $R^4$ are independently selected from hydrogen, alkoxyalkyl, alkyl, haloalkoxyalkyl, and haloalkyl;
$R^5$ is selected from hydrogen, alkyl and haloalkyl;
$R^6$ is selected from phenyl and a five- or six-membered partially or fully unsaturated ring optionally containing one, two, three, or four heteroatoms selected from nitrogen, oxygen, and sulfur; wherein each of the rings is optionally substituted with one, two, three, or four substitutents independently selected from aryl; provided that when $R^6$ is a six-membered substituted ring all substituents on the ring other than fluoro must be in the meta and/or para positions relative to the ring's point of attachment to the parent molecular moiety;
each $R^7$ is independently selected from alkoxy, alkoxycarbonyl, alkyl, alkylcarbonyl, aryl, carboxy, cyano, cyanoalkyl, cycloalkyl, halo, haloalkyl, haloalkoxy, heterocyclyl, hydroxy, hydroxyalkyl, nitro,$-NR^cR^d$, $(NR^cR^d)$alkyl, $(NR^cR^d)$alkoxy, $(NR^eR^f)$carbonyl, and $(NR^eR^f)$sulfonyl; or
two adjacent $R^7$ groups, together with the carbon atoms to which they are attached, form a four- to seven-membered partially- or fully-unsaturated ring optionally containing one or two heteroatoms independently selected from nitrogen, oxygen, and sulfur, wherein the ring is optionally substituted with one, two, or three groups independently selected from alkoxy, alkyl, cyano, halo, haloalkoxy, and haloalkyl;
$R^a$ and $R^b$ are independently selected from hydrogen, alkoxy, alkyl, aryl, arylalkyl, cycloalkyl, (cycloalkyl)alkyl, heterocyclyl, and heterocyclylalkyl; or $R^a$ and $R^b$ together with the nitrogen atom to which they are attached form a four to seven-membered monocyclic heterocyclic ring;
$R^c$ and $R^d$ are independently selected from hydrogen, alkoxyalkyl, alkoxycarbonyl, alkyl, alkylcarbonyl, arylalkyl, and haloalkyl;
$R^e$ and $R^f$ are independently selected from hydrogen, alkyl, aryl, arylalkyl, and heterocyclyl; wherein the aryl, the aryl part of the arylalkyl, and the heterocyclyl are optionally substituted with one or two substituents independently selected from alkoxy, alkyl, and halo; and
$R^g$ and $R^h$ are independently selected from hydrogen, alkyl, aryl, arylalkyl, cycloalkyl, (cycloalkyl)alkyl, heterocyclyl, and heterocyclyl; or $R^g$ and $R^h$ together with the nitrogen atom to which they are attached form a monocyclic heterocyclic ring wherein the monocyclic heterocyclic ring is optionally fused to a phenyl ring to form a bicyclic system; wherein the monocyclic heterocyclic ring and the bicyclic system are optionally substituted with one, two, or three substituents independently selected from alkoxy, alkyl, halo, haloalkoxy, and haloalkyl.

**3.** A compound of claims 1 or 2, or a pharmaceutically acceptable salt thereof, wherein $R^2$ is selected from alkyl and cycloalkyl, wherein the cycloalkyl is optionally substituted with one substituent selected from alkenyl, alkoxy, alkyl, and halo.

**4.** A compound of claims 1 or 2, or a pharmaceutically acceptable salt thereof, wherein $R^3$ and $R^4$ are each hydrogen and Q is a $C_6$ unsaturated chain containing zero heteroatoms.

**5.** A compound of claim 1, or a pharmaceutically acceptable salt thereof, wherein
Q is a $C_6$ unsaturated chain containing zero heteroatoms;
$R^1$ is isoquinolinyl optionally substituted with one or two $R^7$ groups;
$R^2$ is selected from alkyl and cycloalkyl, wherein the cycloalkyl is optionally substituted with one substituent selected from alkenyl, alkoxy, alkyl, and halo;
$R^3$ and $R^4$ are each hydrogen; and
$R^6$ is a six-membered fully unsaturated ring containing one nitrogen atom wherein the ring is optionally substituted with one, two, three, or four substitutents independently selected from alkoxy, alkoxycarbonyl, alkyl, alkylcarbonyl, alkylsulfanyl, carboxy, cyano, cycloalkyl, cycloalkyloxy, halo, haloalkyl, haloalkoxy, $-NR^cR^d$, $(NR^eR^f)$carbonyl, $(NR^eR^f)$sulfonyl, and oxo; provided that all substituents on the ring other than fluoro must be in the meta and/or para positions relative to the ring's point of attachment to the parent molecular moiety.

**6.** A compound of claim 2, or a pharmaceutically acceptable salt thereof, wherein
Q is a $C_6$ unsaturated chain containing zero heteroatoms;
$R^1$ is isoquinolinyl optionally substituted with one or two $R^7$ groups;
$R^2$ is selected from alkyl and cycloalkyl, wherein the cycloalkyl is optionally substituted with one substituent selected from alkenyl, alkoxy, alkyl, and halo;
$R^3$ and $R^4$ are each hydrogen; and
$R^6$ is a six-membered fully unsaturated ring containing one nitrogen atom wherein the ring is optionally substituted with one, two, three, or four substitutents independently selected from aryl; provided that all substituents on the ring other than fluoro must be in the meta and/or para positions relative to the ring's point of attachment to the parent molecular moiety.

**7.** A compound of claim 1 selected from

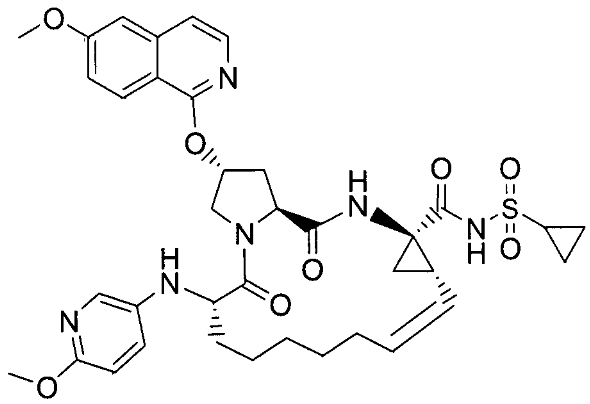

;

and

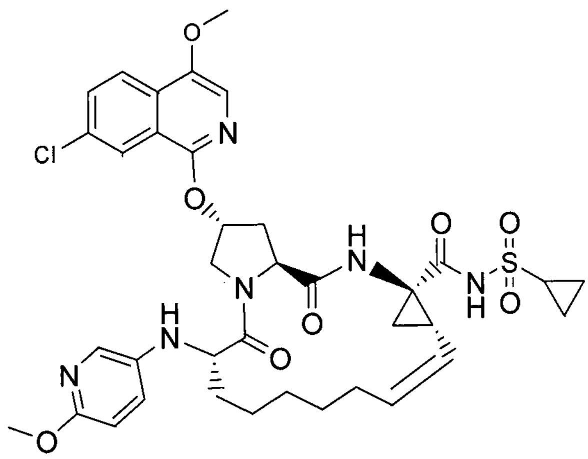

;

or a pharmaceutically acceptable salt thereof.

**8.** A compound of claim 2 selected from

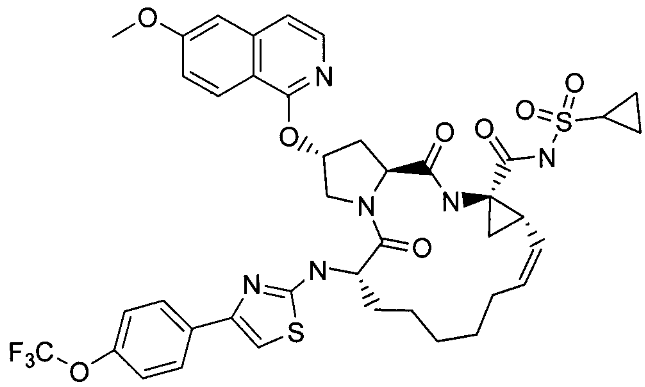

;

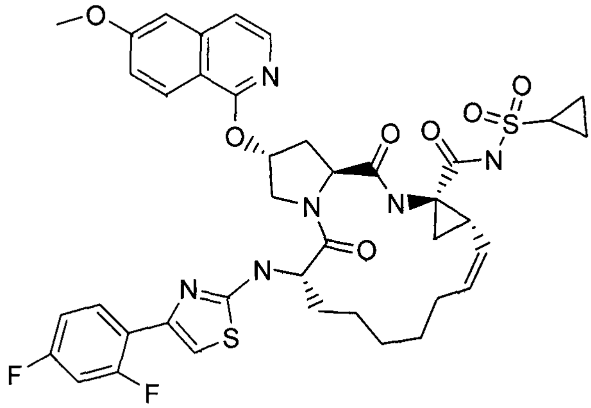

;

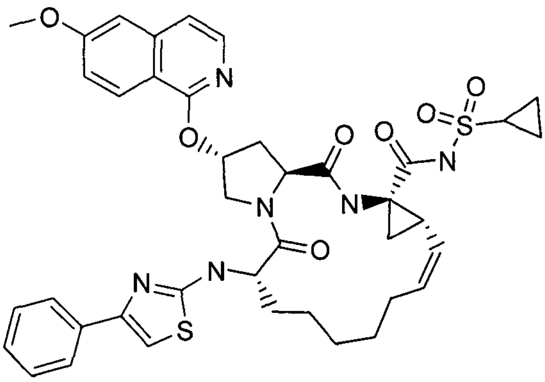

;

and

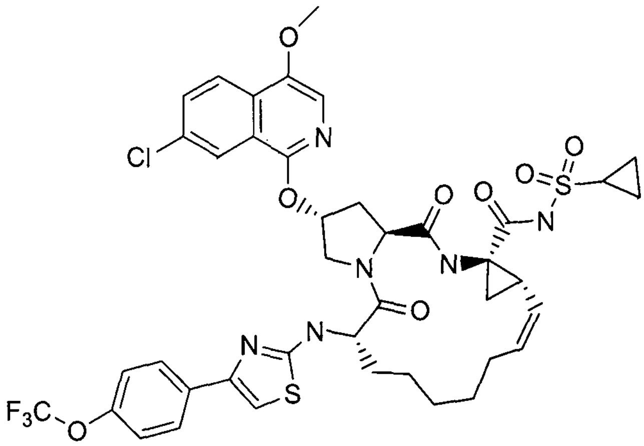

;

or a pharmaceutically acceptable salt thereof.

9. A composition comprising the compound of any one of claims 1 to 8, or a pharmaceutically acceptable salt thereof, and a pharmaceutically acceptable carrier.

10. The composition of claim 9 further comprising at least one additional compound having anti-HCV activity.

11. The composition of claim 10 wherein at least one of the additional compounds is an interferon or a ribavirin.

12. The composition of claim 11 wherein the interferon is selected from interferon alpha 2B, pegylated interferon alpha, consensus interferon, interferon alpha 2A, and lymphoblastiod interferon tau.

13. A compound of any one of claims 1 to 8, or a pharmaceutically acceptable salt thereof for use in treating an HCV infection.

14. The compound of any one of claims 1 to 8, or a pharmaceutically acceptable salt thereof for use according to claim 13 further comprising administering at least one additional compounds having anti-HCV activity prior to, after, or simultaneously with the compound of claim 1, or a pharmaceutically acceptable salt thereof.

15. The compound of any one of claims 1 to 8, or a pharmaceutically acceptable salt thereof for use according to claim 14 wherein at least one of the additional compounds is an interferon or a ribavirin.

16. The compound of any one of claims 1 to 8, or a pharmaceutically acceptable salt thereof for use according to claim 15 wherein the interferon is selected from interferon alpha 2B, pegylated interferon alpha, consensus interferon, interferon alpha 2A, and lymphoblastiod interferon tau.

## Patentansprüche

1. Verbindung der Formel (I):

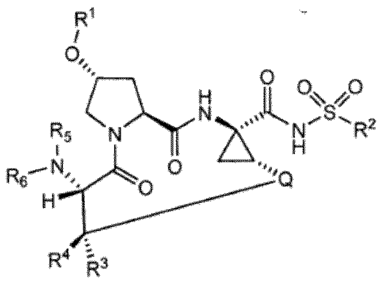

(I),

oder ein pharmazeutisch verträgliches Salz davon, wobei:

Q eine gesättigte oder ungesättigte $C_{3-9}$-Kette ist, die wahlweise ein bis drei Heteroatome enthält, die unabhängig aus O, $S(O)_m$ und $NR^8$ ausgewählt sind; wobei m 0, 1 oder 2 ist und $R^8$ aus Wasserstoff, Alkoxy, Alkoxycarbonyl, Alkyl, Alkylcarbonyl, Alkylsulfonyl, Aminocarbonyl, Arylsulfonyl, Cycloalkyl, (Cycloalkyl)alkyl, Cycloalkyloxy, Dialkylaminocarbonyl, Dialkylaminocarbonylalkyl, Haloalkyl und Heterocyclylcarbonyl ausgewählt ist;

$R^1$ Isochinolinyl ist, das wahlweise mit einer oder zwei $R^7$-Gruppen substituiert ist;

$R^2$ aus Alkyl, Aryl, Cycloalkyl, (Cycloalkyl)alkyl, Heterocyclyl, und $-NR^aR^b$ ausgewählt ist, wobei das Alkyl, das Cycloalkyl und der Cycloalkylteil des (Cycloalkyl)alkyls wahlweise mit einem, zwei oder drei Substituenten substituiert sind, die aus Alkenyl, Alkoxy, Alkoxyalkyl, Alkyl, Arylalkyl, Arylcarbonyl, Cyano, Cycloalkenyl, (Cycloalkyl)alkyl, Halo, Haloalkoxy, Haloalkyl und $(NR^eR^f)$-Carbonyl ausgewählt sind;

$R^3$ und $R^4$ unabhängig aus Wasserstoff, Alkoxyalkyl, Alkyl, Haloalkoxyalkyl und Haloalkyl ausgewählt sind;

$R^5$ aus Wasserstoff, Alkyl und Haloalkyl ausgewählt ist;

$R^6$ aus Phenyl und einem 5- oder 6-gliedrigen teilweise oder vollständig ungesättigten Ring ausgewählt ist, der wahlweise ein, zwei, drei oder vier Heteroatome enthält, die aus Stickstoff, Sauerstoff und Schwefel ausgewählt sind; wobei jeder der Ringe wahlweise mit einem, zwei, drei oder vier Substituenten substituiert ist, die unabhängig aus Alkoxy, Alkoxycarbonyl, Alkyl, Alkylcarbonyl, Alkylsulfanyl, Carboxy, Cyano, Cycloalkyl, Cycloalkyloxy, Halo, Haloalkyl, Haloalkoxy, $-NR^cR^d$, $(NR^eR^f)$-Carbonyl, $(NR^eR^f)$-Sulfonyl und Oxo ausgewählt sind; mit der Maßgabe, dass, wenn $R^6$ ein 6-gliedriger substituierter Ring ist, alle Substituenten am Ring außer Fluor in Bezug auf den Anbindungspunkt des Rings an die Stammmoleküleinheit in den meta- und/oder para-Positionen vorliegen müssen;

$R^7$ jeweils unabhängig aus Alkoxy, Alkoxycarbonyl, Alkyl, Alkylcarbonyl, Aryl, Carboxy, Cyano, Cyanoalkyl, Cycloalkyl, Halo, Haloalkyl, Haloalkoxy, Heterocyclyl, Hydroxy, Hydroxyalkyl, Nitro, $-NR^cR^d$, $(NR^cR^d)$-Alkyl, $(NR^cR^d)$-Alkoxy, $(NR^eR^f)$-Carbonyl und $(NR^eR^f)$-Sulfonyl ausgewählt ist; oder

zwei benachbarte $R^7$-Gruppen zusammen mit den Kohlenstoffatomen, an die sie angebunden sind, einen 4- bis 7-gliedrigen teilweise oder vollständig ungesättigten Ring bilden, der wahlweise ein oder zwei Heteroatome enthält, die unabhängig aus Stickstoff, Sauerstoff und Schwefel ausgewählt sind, wobei der Ring wahlweise mit einer, zwei oder drei Gruppen substituiert ist, die unabhängig aus Alkoxy, Alkyl, Cyano, Halo, Haloalkoxy und Haloalkyl ausgewählt sind;

$R^a$ und $R^b$ unabhängig aus Wasserstoff, Alkoxy, Alkyl, Aryl, Arylalkyl, Cycloalkyl, (Cycloalkyl)alkyl, Heterocyclyl und Heterocyclylalkyl ausgewählt sind; oder $R^a$ und $R^b$ zusammen mit dem Stickstoffatom, an das sie angebunden sind, einen 4- bis 7-gliedrigen monocyclischen heterocyclischen Ring bilden;

$R^c$ und $R^d$ unabhängig aus Wasserstoff, Alkoxyalkyl, Alkoxycarbonyl, Alkyl, Alkylcarbonyl, Arylalkyl und Haloalkyl ausgewählt sind;

$R^e$ und $R^f$ unabhängig aus Wasserstoff, Alkyl, Aryl, Arylalkyl und Heterocyclyl ausgewählt sind; wobei das Aryl, der Arylteil des Arylalkyls und das Heterocyclyl wahlweise mit einem oder zwei Substituenten substituiert sind, die unabhängig aus Alkoxy, Alkyl und Halo ausgewählt sind; und

$R^g$ und $R^h$ unabhängig aus Wasserstoff, Alkyl, Aryl, Arylalkyl, Cycloalkyl, (Cycloalkyl)alkyl, Heterocyclyl und Heterocyclyl ausgewählt sind; oder $R^g$ und $R^h$ zusammen mit dem Stickstoffatom, an das sie angebunden sind, einen monocyclischen heterocyclischen Ring bilden, wobei der monocyclische heterocyclische Ring wahlweise mit einem Phenylring fusioniert ist, um ein bicyclisches System zu bilden; wobei der monocyclische heterocyclische Ring und das bicyclische System wahlweise mit einem, zwei oder drei Substituenten substituiert sind, die unabhängig aus Alkoxy, Alkyl, Halo, Haloalkoxy und Haloalkyl ausgewählt sind.

**2.** Verbindung der Formel (I):

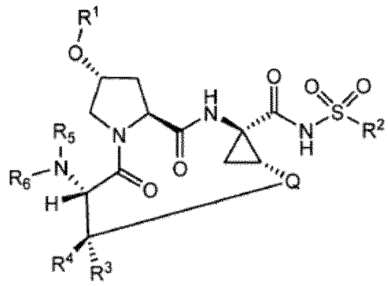

(I),

oder ein pharmazeutisch verträgliches Salz davon, wobei:

Q eine gesättigte oder ungesättigte $C_{3-9}$-Kette ist, die wahlweise ein bis drei Heteroatome enthält, die aus O, $S(O)_m$ und $NR^8$ ausgewählt sind; wobei m 0, 1 oder 2 ist und $R^8$ aus Wasserstoff, Alkoxy, Alkoxycarbonyl, Alkyl, Alkylcarbonyl, Alkylsulfonyl, Aminocarbonyl, Arylsulfonyl, Cycloalkyl, (Cycloalkyl)alkyl, Cycloalkyloxy, Dialkylaminocarbonyl, Dialkylaminocarbonylalkyl, Haloalkyl und Heterocyclylcarbonyl ausgewählt ist;
$R^1$ Isochinolinyl ist, das wahlweise mit einer oder zwei $R^7$-Gruppen substituiert ist;
$R^2$ aus Alkyl, Aryl, Cycloalkyl, (Cycloalkyl)alkyl, Heterocyclyl, und $-NR^aR^b$ ausgewählt ist, wobei das Alkyl, das Cycloalkyl und der Cycloalkylteil des (Cycloalkyl)alkyls wahlweise mit einem, zwei oder drei Substituenten substituiert sind, die aus Alkenyl, Alkoxy, Alkoxyalkyl, Alkyl, Arylalkyl, Arylcarbonyl, Cyano, Cycloalkenyl, (Cycloalkyl)alkyl, Halo, Haloalkoxy, Haloalkyl und $(NR^eR^f)$-Carbonyl ausgewählt sind;
$R^3$ und $R^4$ unabhängig aus Wasserstoff, Alkoxyalkyl, Alkyl, Haloalkoxyalkyl und Haloalkyl ausgewählt sind;
$R^5$ aus Wasserstoff, Alkyl und Haloalkyl ausgewählt ist;
$R^6$ aus Phenyl und einem 5- oder 6-gliedrigen teilweise oder vollständig ungesättigten Ring ausgewählt ist, der wahlweise ein, zwei, drei oder vier Heteroatome enthält, die aus Stickstoff, Sauerstoff und Schwefel ausgewählt sind; wobei jeder der Ringe wahlweise mit einem, zwei, drei oder vier Substituenten substituiert ist, die unabhängig aus Aryl ausgewählt sind; mit der Maßgabe, dass, wenn $R^6$ ein 6-gliedriger substituierter Ring ist, alle Substituenten am Ring außer Fluor in Bezug auf den Anbindungspunkt des Rings an die Stammmoleküleinheit in den meta- und/oder para-Positionen vorliegen müssen;
$R^7$ jeweils unabhängig aus Alkoxy, Alkoxycarbonyl, Alkyl, Alkylcarbonyl, Aryl, Carboxy, Cyano, Cyanoalkyl, Cycloalkyl, Halo, Haloalkyl, Haloalkoxy, Heterocyclyl, Hydroxy, Hydroxyalkyl, Nitro, $(NR^cR^d)$, $(NR^cR^d)$-Alkyl, $(NR^cR^d)$-Alkoxy, $(NR^eR^f)$-Carbonyl und $(NR^eR^f)$-Sulfonyl ausgewählt ist; oder
zwei benachbarte $R^7$-Gruppen zusammen mit den Kohlenstoffatomen, an die sie angebunden sind, einen 4- bis 7-gliedrigen teilweise oder vollständig ungesättigten Ring bilden, der wahlweise ein oder zwei Heteroatome enthält, die unabhängig aus Stickstoff, Sauerstoff und Schwefel ausgewählt sind, wobei der Ring wahlweise mit einer, zwei oder drei Gruppen substituiert ist, die unabhängig aus Alkoxy, Alkyl, Cyano, Halo, Haloalkoxy und Haloalkyl ausgewählt sind;
$R^a$ und $R^b$ unabhängig aus Wasserstoff, Alkoxy, Alkyl, Aryl, Arylalkyl, Cycloalkyl, (Cycloalkyl)alkyl, Heterocyclyl und Heterocyclylalkyl ausgewählt sind; oder $R^a$ und $R^b$ zusammen mit dem Stickstoffatom, an das sie angebunden sind, einen 4- bis 7-gliedrigen monocyclischen heterocyclischen Ring bilden;
$R^c$ und $R^d$ unabhängig aus Wasserstoff, Alkoxyalkyl, Alkoxycarbonyl, Alkyl, Alkylcarbonyl, Arylalkyl und Haloalkyl ausgewählt sind;
$R^e$ und $R^f$ unabhängig aus Wasserstoff, Alkyl, Aryl, Arylalkyl und Heterocyclyl ausgewählt sind; wobei das Aryl, der Arylteil des Arylalkyls und das Heterocyclyl wahlweise mit einem oder zwei Substituenten substituiert sind, die unabhängig aus Alkoxy, Alkyl und Halo ausgewählt sind; und
$R^g$ und $R^h$ unabhängig aus Wasserstoff, Alkyl, Aryl, Arylalkyl, Cycloalkyl, (Cycloalkyl)alkyl, Heterocyclyl und Heterocyclyl ausgewählt sind; oder $R^g$ und $R^h$ zusammen mit dem Stickstoffatom, an das sie angebunden sind, einen monocyclischen heterocyclischen Ring bilden, wobei der monocyclische heterocyclische Ring wahlweise mit einem Phenylring fusioniert ist, um ein bicyclisches System zu bilden; wobei der monocyclische heterocyclische Ring und das bicyclische Ringsystem wahlweise mit einem, zwei oder drei Substituenten substituiert

sind, die unabhängig aus Alkoxy, Alkyl, Halo, Haloalkoxy und Haloalkyl ausgewählt sind.

**3.** Verbindung nach Anspruch 1 oder 2 oder ein pharmazeutisch verträgliches Salz davon, wobei $R^2$ aus Alkyl und Cycloalkyl ausgewählt ist, wobei das Cycloalkyl wahlweise mit einem Substituenten substituiert ist, der aus Alkenyl, Alkoxy, Alkyl und Halo ausgewählt ist.

**4.** Verbindung nach Anspruch 1 oder 2 oder ein pharmazeutisch verträgliches Salz davon, wobei $R^3$ und $R^4$ jeweils Wasserstoff sind und Q eine ungesättigte $C_6$-Kette ist, die null Heteroatome enthält.

**5.** Verbindung nach Anspruch 1 oder ein pharmazeutisch verträgliches Salz davon, wobei:

Q eine ungesättigte $C_6$-Kette ist, die null Heteroatome enthält;
$R^1$ Isochinolinyl ist, das wahlweise mit einer oder zwei $R^7$-Gruppen substituiert ist;
$R^2$ aus Alkyl und Cycloalkyl ausgewählt ist, wobei das Cycloalkyl wahlweise mit einem Substituenten substituiert ist, der aus Alkenyl, Alkoxy, Alkyl und Halo ausgewählt ist;
$R^3$ und $R^4$ jeweils Wasserstoff sind; und
$R^6$ ein 6-gliedriger vollständig ungesättigter Ring ist, der ein Stickstoffatom enthält, wobei der Ring wahlweise mit einem, zwei, drei oder vier Substituenten substituiert ist, die unabhängig aus Alkoxy, Alkoxycarbonyl, Alkyl, Alkylcarbonyl, Alkylsulfanyl, Carboxy, Cyano, Cycloalkyl, Cycloalkyloxy, Halo, Haloalkyl, Haloalkoxy, $-NR^cR^d$, $(NR^eR^f)$-Carbonyl, $(NR^eR^f)$-Sulfonyl und Oxo ausgewählt sind; mit der Maßgabe, dass alle Substituenten am Ring außer Fluor in Bezug auf den Anbindungspunkt des Rings an die Stammmoleküleinheit in den meta- und/oder para-Positionen vorliegen müssen.

**6.** Verbindung nach Anspruch 2 oder ein pharmazeutisch verträgliches Salz davon, wobei:

Q eine ungesättigte $C_6$-Kette ist, die null Heteroatome enthält;
$R^1$ Isochinolinyl ist, das wahlweise mit einer oder zwei $R^7$-Gruppen substituiert ist;
$R^2$ aus Alkyl und Cycloalkyl ausgewählt ist, wobei das Cycloalkyl wahlweise mit einem Substituenten substituiert ist, der aus Alkenyl, Alkoxy, Alkyl und Halo ausgewählt ist;
$R^3$ und $R^4$ jeweils Wasserstoff sind; und
$R^6$ ein 6-gliedriger vollständig ungesättigter Ring ist, der ein Stickstoffatom enthält, wobei der Ring wahlweise mit einem, zwei, drei oder vier Substituenten substituiert ist, die unabhängig aus Aryl ausgewählt sind; mit der Maßgabe, dass alle Substituenten am Ring außer Fluor in Bezug auf den Anbindungspunkt des Rings an die Stammmoleküleinheit in den meta- und/oder para-Positionen vorliegen müssen.

**7.** Verbindung nach Anspruch 1, ausgewählt aus:

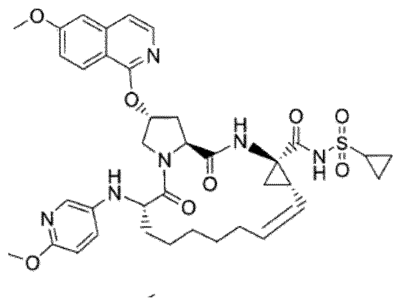
;

und

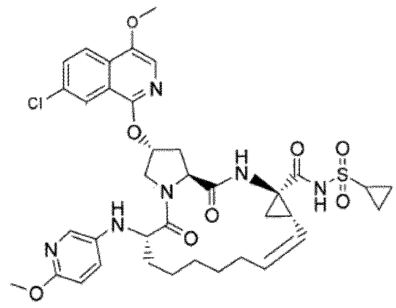

;

oder einem pharmazeutisch verträglichen Salz davon.

**8.** Verbindung nach Anspruch 2, ausgewählt aus:

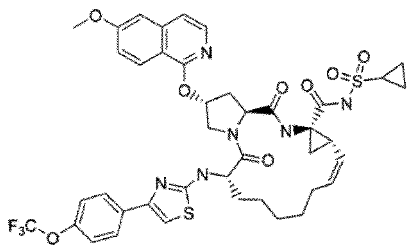

;

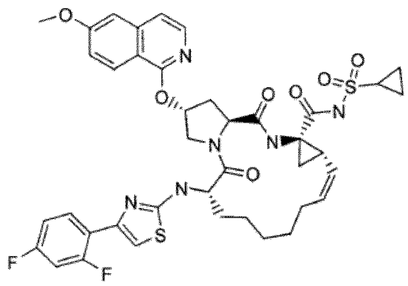

;

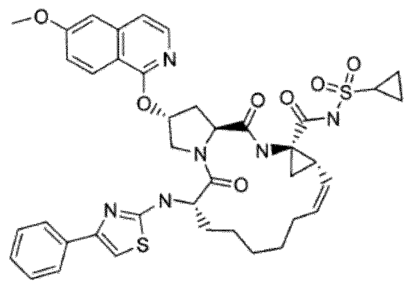

;

und

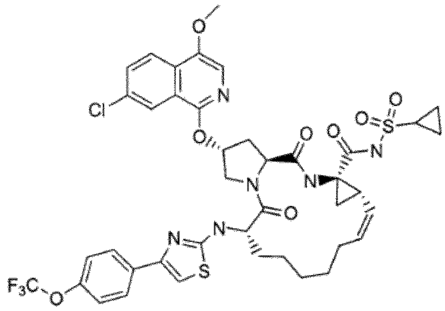

;

oder einem pharmazeutisch verträglichen Salz davon.

9. Zusammensetzung, die die Verbindung nach einem der Ansprüche 1 bis 8 oder ein pharmazeutisch verträgliches Salz davon und einen pharmazeutisch verträglichen Träger umfasst.

10. Zusammensetzung nach Anspruch 9, die des Weiteren zumindest eine zusätzliche Verbindung mit Anti-HCV-Aktivität umfasst.

11. Zusammensetzung nach Anspruch 10, wobei zumindest eine der zusätzlichen Verbindungen ein Interferon oder ein Ribavirin ist.

12. Zusammensetzung nach Anspruch 11, wobei das Interferon aus Interferon alpha 2B, pegyliertem Interferon alpha, Konsensus-Interferon, Interferon alpha 2A und lymphoblastoidem Interferon tau ausgewählt ist.

13. Verbindung nach einem der Ansprüche 1 bis 8 oder ein pharmazeutisch verträgliches Salz davon zur Verwendung bei der Behandlung einer HCV-Infektion.

14. Verbindung nach einem der Ansprüche 1 bis 8 oder ein pharmazeutisch verträgliches Salz davon zur Verwendung nach Anspruch 13, des Weiteren umfassend das Verabreichen zumindest einer zusätzlichen Verbindung mit einer Anti-HCV-Aktivität vor, nach oder gleichzeitig mit der Verbindung nach Anspruch 1 oder eines pharmazeutisch verträglichen Salzes davon.

15. Verbindung nach einem der Ansprüche 1 bis 8 oder ein pharmazeutisch verträgliches Salz davon zur Verwendung nach Anspruch 14, wobei zumindest eine der zusätzlichen Verbindungen ein Interferon oder ein Ribavirin ist.

16. Verbindung nach einem der Ansprüche 1 bis 8 oder ein pharmazeutisch verträgliches Salz davon zur Verwendung nach Anspruch 15, wobei das Interferon aus Interferon alpha 2B, pegyliertem Interferon alpha, Konsensus-Interferon, Interferon alpha 2A und lymphoblastoidem Interferon tau ausgewählt ist.

## Revendications

1. Composé de la formule (I):

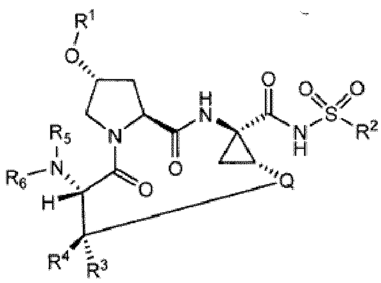

(I),

ou un de ses sels pharmaceutiquement acceptables, dans lequel:

Q est une chaîne $C_{3-9}$ saturée ou insaturée contenant optionnellement de un à trois hétéroatomes indépendamment choisis parmi O, $S(O)_m$ et $NR^8$; où m est 0, 1 ou 2 et $R^8$ est choisi parmi un hydrogène, un alcoxy, un alcoxycarbonyle, un alkyle, un alkylcarbonyle, un alkylsulfonyle, un aminocarbonyle, un arylsulfonyle, un cycloalkyle, un (cycloalkyl)alkyle, un cycloalkyloxy, un dialkylaminocarbonyle, un dialkylaminocarbonylalkyle, un haloalkyle et un hétérocyclylcarbonyle;
$R^1$ est un isoquinoléinyle optionnellement substitué avec un ou deux groupes $R^7$;
$R^2$ est choisi parmi un alkyle, un aryle, un cycloalkyle, un (cycloalkyl)alkyle, un hétérocyclyle et $-NR^aR^b$, où l'alkyle, le cycloalkyle et la partie cycloalkyle du (cycloalkyl)alkyle sont optionnellement substitués avec un, deux ou trois substituants choisis parmi un alcényle, un alcoxy, un alcoxyalkyle, un alkyle, un arylalkyle, un arylcarbonyle, un cyano, un cycloalcényle, un (cycloalkyl)alkyle, un halo, un haloalcoxy, un haloalkyle et un $(NR^eR^f)$carbonyle;
$R^3$ et $R^4$ sont indépendamment choisis parmi un hydrogène, un alcoxyalkyle, un alkyle, un haloalcoxyalkyle et un haloalkyle;
$R^5$ est choisi parmi un hydrogène, un alkyle et un haloalkyle;
$R^6$ est choisi parmi un phényle et un cycle de cinq ou six membres partiellement ou totalement insaturé contenant optionnellement un, deux, trois ou quatre hétéroatomes choisis parmi un azote, un oxygène et un soufre; où chacun des cycles est optionnellement substitué avec un, deux, trois ou quatre substituants indépendamment choisis parmi un alcoxy, un alcoxycarbonyle, un alkyle, un alkylcarbonyle, un alkylsulfanyle, un carboxy, un cyano, un cycloalkyle, un cycloalkyloxy, un halo, un haloalkyle, un haloalcoxy, $-NR^cR^d$, un $(NR^eR^f)$carbonyle, un $(NR^eR^f)$sulfonyle et un oxo; à condition que lorsque $R^6$ est un cycle de six membres substitué, tous les substituants sur le cycle autres que le fluoro doivent être aux positions méta et/ou para relativement au point d'attache du cycle avec la fraction moléculaire parente;
chaque $R^7$ est indépendamment choisi parmi un alcoxy, un alcoxycarbonyle, un alkyle, un alkylcarbonyle, un aryle, un carboxy, un cyano, un cyanoalkyle, un cycloalkyle, un halo, un haloalkyle, un haloalcoxy, un hétérocyclyle, un hydroxy, un hydroxyalkyle, un nitro, $-NR^cR^d$, un $(NR^cR^d)$alkyle, un $(NR^cR^d)$alcoxy, un $(NR^eR^f)$carbonyle et un $(NR^eR^f)$sulfonyle; ou
deux groupes $R^7$ adjacents, accompagnés des atomes de carbone auxquels ils sont attachés, forment un cycle de quatre à sept membres partiellement ou totalement insaturé contenant optionnellement un ou deux hétéroatomes indépendamment choisis parmi un azote, un oxygène et un soufre, où le cycle est optionnellement substitué avec un, deux ou trois groupes indépendamment choisis parmi un alcoxy, un alkyle, un cyano, un halo, un haloalcoxy et un haloalkyle;
$R^a$ et $R^b$ sont indépendamment choisis parmi un hydrogène, un alcoxy, un alkyle, un aryle, un arylalkyle, un cycloalkyle, un (cycloalkyl)alkyle, un hétérocyclyle et un hétérocyclylalkyle; ou $R^a$ et $R^b$ accompagnés de l'atome d'azote auquel ils sont attachés forment un cycle hétérocyclique de quatre à sept membres monocyclique;
$R^c$ et $R^d$ sont indépendamment choisis parmi un hydrogène, un alcoxyalkyle, un alcoxycarbonyle, un alkyle, un alkylcarbonyle, un arylalkyle et un haloalkyle;
$R^e$ et $R^f$ sont indépendamment choisis parmi un hydrogène, un alkyle, un aryle, un arylalkyle et un hétérocyclyle; où l'aryle, la partie aryle de l'arylalkyle et l'hétérocyclyle sont optionnellement substitués avec un ou deux substituants indépendamment choisis parmi un alcoxy, un alkyle et un halo; et
$R^g$ et $R^h$ sont indépendamment choisis parmi un hydrogène, un alkyle, un aryle, un arylalkyle, un cycloalkyle, un (cycloalkyl)alkyle, un hétérocyclyle et un hétérocyclyle; ou $R^g$ et $R^h$ accompagnés de l'atome d'azote auquel ils sont attachés forment un cycle hétérocyclique monocyclique où le cycle hétérocyclique monocyclique est optionnellement condensé à un cycle de phényle pour former un système bicyclique; où le cycle hétérocyclique monocyclique et le système bicyclique sont optionnellement substitués avec un, deux ou trois substituants indépendamment choisis parmi un alcoxy, un alkyle, un halo, un haloalcoxy et un haloalkyle.

**2.** Composé de la formule (I):

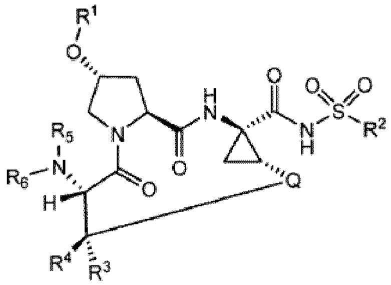

(I),

ou un de ses sels pharmaceutiquement acceptables, dans lequel:

Q est une chaîne $C_{3-9}$ saturée ou insaturée contenant optionnellement de un à trois hétéroatomes indépendamment choisis parmi O, $S(O)_m$ et $NR^8$; où m est 0, 1 ou 2 et $R^8$ est choisi parmi un hydrogène, un alcoxy, un alcoxycarbonyle, un alkyle, un alkylcarbonyle, un alkylsulfonyle, un aminocarbonyle, un arylsulfonyle, un cycloalkyle, un (cycloalkyl)alkyle, un cycloalkyloxy, un dialkylaminocarbonyle, un dialkylaminocarbonylalkyle, un haloalkyle et un hétérocyclylcarbonyle;

$R^1$ est un isoquinoléinyle optionnellement substitué avec un ou deux groupes $R^7$;

$R^2$ est choisi parmi un alkyle, un aryle, un cycloalkyle, un (cycloalkyl)alkyle, un hétérocyclyle et $-NR^aR^b$, où l'alkyle, le cycloalkyle et la partie cycloalkyle du (cycloalkyl)alkyle sont optionnellement substitués avec un, deux ou trois substituants choisis parmi un alcényle, un alcoxy, un alcoxyalkyle, un alkyle, un arylalkyle, un arylcarbonyle, un cyano, un cycloalcényle, un (cycloalkyl)alkyle, un halo, un haloalcoxy, un haloalkyle et un $(NR^eR^f)$carbonyle;

$R^3$ et $R^4$ sont indépendamment choisis parmi un hydrogène, un alcoxyalkyle, un alkyle, un haloalcoxyalkyle et un haloalkyle;

$R^5$ est choisi parmi un hydrogène, un alkyle et un haloalkyle;

$R^6$ est choisi parmi un phényle et un cycle de cinq ou six membres partiellement ou totalement insaturé contenant optionnellement un, deux, trois ou quatre hétéroatomes choisis parmi un azote, un oxygène et un soufre; où chacun des cycles est optionnellement substitué avec un, deux, trois ou quatre substituants indépendamment choisis parmi un aryle; à condition que lorsque $R^6$ est un cycle de six membres substitué, tous les substituants sur le cycle autres que le fluoro doivent être aux positions méta et/ou para relativement au point d'attache du cycle avec la fraction moléculaire parente;

chaque $R^7$ est indépendamment choisi parmi un alcoxy, un alcoxycarbonyle, un alkyle, un alkylcarbonyle, un aryle, un carboxy, un cyano, un cyanoalkyle, un cycloalkyle, un halo, un haloalkyle, un haloalcoxy, un hétérocyclyle, un hydroxy, un hydroxyalkyle, un nitro, $-NR^cR^d$, un $(NR^cR^d)$alkyle, un $(NR^cR^d)$alcoxy, un $(NR^eR^f)$carbonyle et un $(NR^eR^f)$sulfonyle; ou

deux groupes $R^7$ adjacents, accompagnés des atomes de carbone auxquels ils sont attachés, forment un cycle de quatre à sept membres partiellement ou totalement insaturé contenant optionnellement un ou deux hétéroatomes indépendamment choisis parmi un azote, un oxygène et un soufre, où le cycle est optionnellement substitué avec un, deux ou trois groupes indépendamment choisis parmi un alcoxy, un alkyle, un cyano, un halo, un haloalcoxy et un haloalkyle;

$R^a$ et $R^b$ sont indépendamment choisis parmi un hydrogène, un alcoxy, un alkyle, un aryle, un arylalkyle, un cycloalkyle, un (cycloalkyl)alkyle, un hétérocyclyle et un hétérocyclylalkyle; ou $R^a$ et $R^b$ accompagnés de l'atome d'azote auquel ils sont attachés forment un cycle hétérocyclique de quatre à sept membres monocyclique;

$R^c$ et $R^d$ sont indépendamment choisis parmi un hydrogène, un alcoxyalkyle, un alcoxycarbonyle, un alkyle, un alkylcarbonyle, un arylalkyle et un haloalkyle;

$R^e$ et $R^f$ sont indépendamment choisis parmi un hydrogène, un alkyle, un aryle, un arylalkyle et un hétérocyclyle; où l'aryle, la partie aryle de l'arylalkyle et l'hétérocyclyle sont optionnellement substitués avec un ou deux substituants indépendamment choisis parmi un alcoxy, un alkyle et un halo; et

$R^g$ et $R^h$ sont indépendamment choisis parmi un hydrogène, un alkyle, un aryle, un arylalkyle, un cycloalkyle, un (cycloalkyl)alkyle, un hétérocyclyle et un hétérocyclyle; ou $R^g$ et $R^h$ accompagnés de l'atome d'azote auquel ils sont attachés forment un cycle hétérocyclique monocyclique où le cycle hétérocyclique monocyclique est optionnellement condensé à un cycle de phényle pour former un système bicyclique; où le cycle hétérocyclique monocyclique et le système bicyclique sont optionnellement substitués avec un, deux ou trois substituants indépendamment choisis parmi un alcoxy, un alkyle, un halo, un haloalcoxy et un haloalkyle.

3. Composé selon la revendication 1 ou 2, ou un de ses sels pharmaceutiquement acceptables, dans lequel $R^2$ est choisi parmi un alkyle et un cycloalkyle, dans lequel le cycloalkyle est optionnellement substitué avec un substituant choisi parmi un alcényle, un alcoxy, un alkyle et un halo.

4. Composé selon la revendication 1 ou 2, ou un de ses sels pharmaceutiquement acceptables, dans lequel $R^3$ et $R^4$ sont chacun un hydrogène et Q est une chaîne $C_6$ insaturée contenant zéro hétéroatome.

5. Composé selon la revendication 1, ou un de ses sels pharmaceutiquement acceptables, dans lequel:

Q est une chaîne $C_6$ insaturée contenant zéro hétéroatome;
$R^1$ est un isoquinoléinyle optionnellement substitué avec un ou deux groupes $R^7$;
$R^2$ est choisi parmi un alkyle et un cycloalkyle, où le cycloalkyle est optionnellement substitué avec un substituant choisi parmi un alcényle, un alcoxy, un alkyle et un halo;
$R^3$ et $R^4$ sont chacun un hydrogène; et
$R^6$ est un cycle de six membres totalement insaturé contenant un atome d'azote;

où le cycle est optionnellement substitué avec un, deux, trois ou quatre substituants indépendamment choisis parmi un alcoxy, un alcoxycarbonyle, un alkyle, un alkylcarbonyle, un alkylsulfanyle, un carboxy, un cyano, un cycloalkyle, un cycloalkyloxy, un halo, un haloalkyle, un haloalcoxy, $-NR^cR^d$, un $(NR^eR^f)$carbonyle, un $(NR^eR^f)$sulfonyle et un oxo; à condition que tous les substituants sur le cycle autres que le fluoro doivent être aux positions méta et/ou para relativement au point d'attache du cycle avec la fraction moléculaire parente.

6. Composé selon la revendication 2, ou un de ses sels pharmaceutiquement acceptables, dans lequel:

Q est une chaîne $C_6$ insaturée contenant zéro hétéroatome;
$R^1$ est un isoquinoléinyle optionnellement substitué avec un ou deux groupes $R^7$;
$R^2$ est choisi parmi un alkyle et un cycloalkyle, où le cycloalkyle est optionnellement substitué avec un substituant choisi parmi un alcényle, un alcoxy, un alkyle et un halo;
$R^3$ et $R^4$ sont chacun un hydrogène; et
$R^6$ est un cycle de six membres totalement insaturé contenant un atome d'azote;

où le cycle est optionnellement substitué avec un, deux, trois ou quatre substituants indépendamment choisis parmi un aryle; à condition que tous les substituants sur le cycle autres que le fluoro doivent être aux positions méta et/ou para relativement au point d'attache du cycle avec la fraction moléculaire parente.

7. Composé selon la revendication 1 choisi parmi:

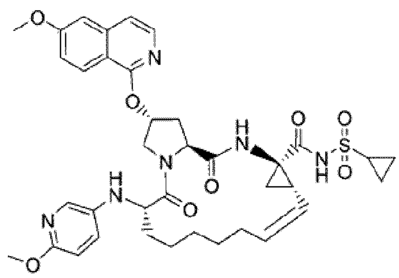
;

et

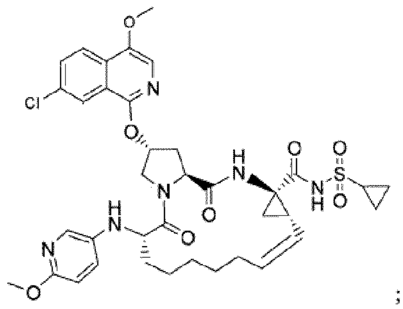

;

ou un de leurs sels pharmaceutiquement acceptables.

**8.** Composé selon la revendication 2 choisi parmi:

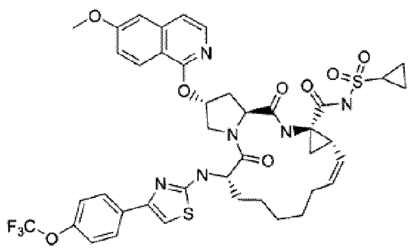

;

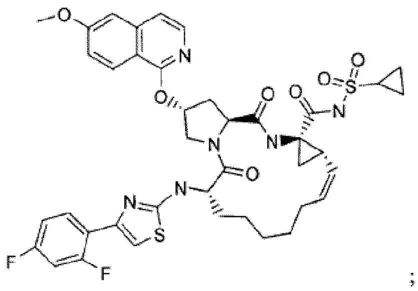

;

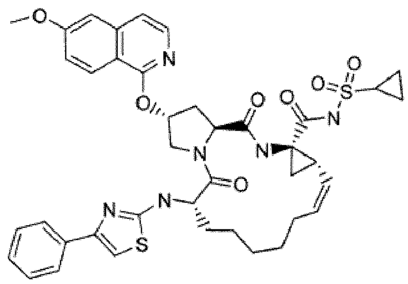

;

et

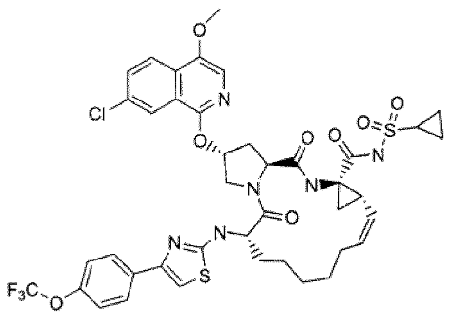

;

ou un de leurs sels pharmaceutiquement acceptables.

**9.** Composition comprenant le composé selon l'une quelconque des revendications 1 à 8, ou un de ses sels pharmaceutiquement acceptables, et un support pharmaceutiquement acceptable.

**10.** Composition selon la revendication 9 comprenant en outre au moins un composé supplémentaire ayant une activité anti-VHC.

**11.** Composition selon la revendication 10, dans laquelle au moins un des composés supplémentaires est un interféron ou une ribavirine.

**12.** Composition selon la revendication 11, dans laquelle l'interféron est choisi parmi un interféron alpha 2B, un interféron alpha pégylé, un interféron consensus, un interféron alpha 2A et un interféron tau lymphoblastoïde.

**13.** Composé selon l'une quelconque des revendications 1 à 8, ou un de ses sels pharmaceutiquement acceptables, pour une utilisation dans le traitement d'une infection par VHC.

**14.** Composé selon l'une quelconque des revendications 1 à 8, ou un de ses sels pharmaceutiquement acceptables, pour une utilisation selon la revendication 13 comprenant en outre l'administration d'au moins un composé supplémentaire ayant une activité anti-VHC avant, après ou simultanément au composé selon la revendication 1, ou à un de ses sels pharmaceutiquement acceptables.

**15.** Composé selon l'une quelconque des revendications 1 à 8, ou un de ses sels pharmaceutiquement acceptables, pour une utilisation selon la revendication 14, dans lequel au moins un des composés supplémentaires est un interféron ou une ribavirine.

**16.** Composé selon l'une quelconque des revendications 1 à 8, ou un de ses sels pharmaceutiquement acceptables, pour une utilisation selon la revendication 15, dans lequel l'interféron est choisi parmi un interféron alpha 2B, un interféron alpha pégylé, un interféron consensus, un interféron alpha 2A et un interféron tau lymphoblastoïde.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007015824 A
- WO 2007056120 A
- WO 2004094452 A
- WO 2008021960 A
- WO 2008137779 A
- WO 2005047288 A
- WO 0009543 A


### Non-patent literature cited in the description


- Remington's Pharmaceutical Sciences. Mack Publishing Company, 1999
- *Pharmaceutical Research,* 1986, vol. 3 (6), 318
- **NICOLAS BRIET et al.** *Tetrahedron,* 2002, 5761-5766
- **P SIMMONDS ; KA ROSE ; S GRAHAM ; SW CHAN ; F MCOMISH ; BC DOW ; EA FOLLETT ; PL YAP ; H MARSDEN.** *J. Clin. Microbiol.,* 1993, vol. 31 (6), 1493-1503
- **YANAGI,M. ; PURCELL,R.H. ; EMERSON,S.U. ; BUKH,J.** *Proc. Natl. Acad. Sci. U.S.A.,* 1997, vol. 94 (16), 8738-8743
- **YANAGI,M. ; ST CLAIRE,M. ; SHAPIRO,M. ; EMERSON,S.U. ; PURCELL,R.H. ; BUKH.** *J, Virology,* 1998, vol. 244 (1), 161-172
- **GALLINARI P ; PAOLINI C ; BRENNAN D ; NARDI C ; STEINKUHLER C ; DE FRANCESCO R.** *Biochemistry,* 1999, vol. 38 (17), 5620-32
- **GALLINARI P ; BRENNAN D ; NARDI C ; BRUNETTI M ; TOMEI L ; STEINKUHLER C ; DE FRANCESCO R.** *J Virol.,* 1998, vol. 72 (8), 6758-69
- **TALIANI et al.** *Anal. Biochem.,* 1996, vol. 240 (2), 60-67
- **LOHMANN V ; KORNER F ; KOCH J ; HERIAN U ; THEILMANN L ; BARTENSCHLAGER R.** *Science,* 1999, vol. 285 (5424), 110-3
- **KRIEGER N ; LOHMANN V ; BARTENSCHLAGER R.** *J. Virol.,* 2001, vol. 75 (10), 4614-4624
- **BLIGHT KJ ; KOLYKHALOV, AA ; RICE, CM.** *Science,* vol. 290 (5498), 1972-1974